# EUROPEAN PATENT SPECIFICATION

(11) **EP 1 848 793 B1**
(45) Date of publication and mention of the grant of the patent: **20.06.2012**
(21) Application number: 06735246.8
(22) Date of filing: 15.02.2006
(51) Int. Cl.: C12M 3/00, C12Q 1/68, B01D 15/36

(54) **POLYELECTROLYTE-COATED ION-EXCHANGE PARTICLES**
POLYELEKTROLYT-BESCHICHTETE IONENAUSTAUSCHTEILCHEN
PARTICULES D'ECHANGE IONIQUE REVETUES DE POLYELECTROLYTES

(30) Priority: 15.02.2005 US 57936; 18.08.2005 US 709986 P
(43) Date of publication of application: 31.10.2007
(73) Proprietor: Life Technologies Corporation, Carlsbad, CA 92008 (US)
(72) Inventor: HARROLD, Michael P., San Mateo, California 94402 (US); LAU, Aldrich N.K., Palo Alto, California 94301 (US); JOHNSON, Ben F., Palo Alto, California 94303 (US); AMPARO, Gilbert P., Hayward, California 94542 (US); MERCER, Frank W., Belmont, California 94002 (US)
(74) Representative: HOFFMANN EITLE
(86) International application number: PCT/US2006/005489
(87) International publication number: WO 2006/089029

(56) References cited:
- WO-A1-2004/011141
- WO-A1-2005/079984
- WO-A2-01/94574
- US-B2- 6 504 021

## Description

### FIELD

The present invention relates to solutions comprising coated particles and methods using same for filtering and/or purifying a sample by using ion-exchange techniques.

### BACKGROUND

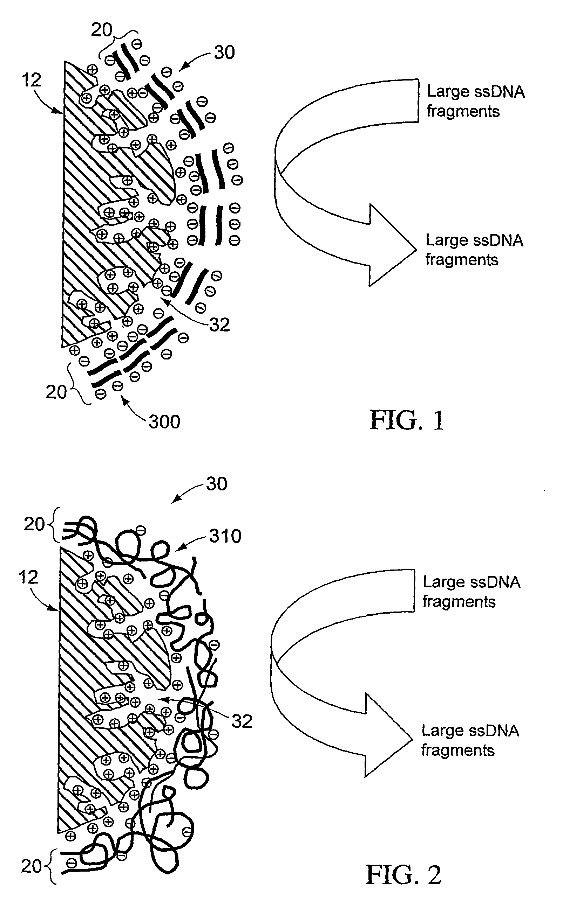

Purification of reaction products obtained from, for example, a polymerase chain reaction (PCR) or a sequencing reaction, can present a number of challenges for subsequent, downstream processing. Impurities can cause artifacts in subsequent processing steps. Numerous purification steps to eliminate artifacts can be cumbersome and inefficient. It can be desirable to capture primers, unincorporated nucleotides, primer-dimers, small DNA fragments, and in some cases desalt PCR products. It can be desirable to capture primers, dye-labeled primers, nucleotides, dye-labeled nucleotides, dideoxynucleotides, and dye labeled dideoxynucleotides and desalt DNA sequencing reaction products. A need exists for separation that addresses these and other problems associated with conventional techniques of purification.
WO 2004/011141 A1 describes a size-exclusion ion-exchange particle and a device using the particle wherein the particle includes an ion-exchange core micro-encapsulated by a shell, and the shell includes a polymerization product of a reactive monomer. The shell can be an at least partially cross-linked polymer. The shell can be capable of excluding molecules of a size equal to or larger than a 10 nt ssDNA molecule or molecules of a size equal to or larger than a 100 nt ssDNA molecule, for example, and the core can be capable of ion-exchange. Methods of making size-exclusion ion-exchange particles are also described, as are methods of purification using the particles.
WO 01/94574 A2 describes a method and an apparatus useful for purifying DNA sequencing reaction products. Briefly, a gel filtration medium is combined with a molecular cutoff filter in a single apparatus to isolate DNA sequencing fragments from the sequencing template, enzyme, salt and nucleotides. A preferred embodiment is that of Figure 1 which shows a cylindrical housing having openings at the top and bottom of the housing. The housing may take forms other than cylindrical, e.g., rectangular, octagonal, etc. The apparatus may be used in conjunction with pressure and/or centrifugation to achieve the separation, and the addition of a detergent composition (nonionic, ionic, or zwitterionic), including a bile salt, may also be used.

### SUMMARY

The present invention relates to solutions comprising coated particles and methods using these solutions to filter and/or purify a sample by ion-exchange reaction as defined in the claims. In particular, the invention relates to:

A solution comprising: a particle, the particle comprising: a core comprising ion-exchange material; and a coating covering the outer surface of the ion-exchange core wherein the coating comprises polyelectrolyte material, wherein the core and coating are adapted to separated DNA sequencing reaction products and wherein the polyelectrolyte creates a size exclusion barrier allowing small molecules which have a size of less than 300 bp to penetrate into the particle and restricting larger molecules from interacting with the core; a non-ionic surfactant; and a stabilizing additive which stabilizes DNA sequencing reactions in a deionized aqueous solution and which is a non-ionic material that is not removed from solution by the polyelectrolyte-coated ion-exchange particle.

A method for DNA sequencing, the method comprising: providing a purification media, contacting the purification media comprising the solution of claim 1 with DNA sequencing reaction products to remove unincorporated dye-terminators and salts; isolating the purification media; and loading a sample for capillary electrophoresis from the DNA sequencing products without interference from the purification media.

Additional features and advantages of various embodiments will be set forth in part in the description that follows, and in part will be apparent from the description, or may be learned by practice of various embodiments. The objectives and other advantages of various embodiments will be realized and attained by means of the elements and combinations particularly pointed out in the description herein and appended claims.

### BRIEF DESCRIPTION OF THE FIGURES

Fig. 1 illustrates a cross-sectional view of a polyelectrolyte-coated particle, where the coating includes a biopolymer;

Fig. 2 illustrates a cross-sectional view of a polyelectrolyte-coated particle, where the coating is a synthetic polymer;

Fig. 2a illustrates several synthetic polymers that can be included in the coating for the polyelectrolyte-coated particle.

Figs. 3a-3d demonstrate separation of sequencing reaction products with polyelectrolyte-coated particles with biopolymer in comparison with standard separation techniques, where Figs. 3a-3c demonstrate separation with polyelectrolyte-coated particles, according to various embodiments, Fig. 3d demonstrates separation with an uncoated ion-exchange particle;

Figs. 4a-4b demonstrate separation of PCR reaction products by polyelectrolyte-coated particles with biopolymer, where Fig. 4a illustrates unpurified PCR reaction products including a mixture of a dye-labeled amplicon and a dye-labeled primer, and Fig. 4b illustrates PCR reaction products separated with a polyelectrolyte-coated particle to remove the dye-labeled primer;

Figs. 5a-5b is a set of graphs illustrating a detail of Figs. 4a-4b, respectively;

Fig. 6 demonstrates separation of a sequencing reaction products with polyelectrolyte-coated particles with synthetic polymer;

Figs. 7a-7b demonstrate separation of a sequencing reaction products with polyelectrolyte-coated particles synthetic polymer;

Fig. 8 demonstrates the size cutoff for separation by the polyelectrolyte-coated particles with synthetic polymer for separation using coating polymers with different molecular weights;

Figs. 9a and 9b demonstrate the separation of sequencing reaction products by polyelectrolyte-coated particles with synthetic polymer,

Fig. 10 demonstrates the size-based removal of small dsDNA fragments from larger dsDNA fragments using polyelectrolyte-coated particles with synthetic polymer,

Fig. 11 demonstrates the removal of an oligonucleotide primer from a PCR product using polyelectrolyte-coated particles by illustrating the result of separating components with gel electrophoresis using a 2% agarose gel; and

Fig. 12 demonstrates the DNA size discrimination using non-desalting polyelectrolyte-coated particles. Figs. 13-24 demonstrate additional examples.

It is to be understood that the figures are not drawn to scale. Further, the relation between objects in a figure may not be to scale, and may in fact have a reverse relationship as to size. The figures are intended to bring understanding and clarity to the structure of each object shown, and thus, some features may be exaggerated in order to illustrate a specific feature of a structure.

It is to be understood that both the foregoing general description and the following detailed description are exemplary and explanatory only, and are intended to provide an explanation of various embodiments of the present teachings.

### DESCRIPTION OF VARIOUS EMBODIMENTS

The term "particle" as used herein refers to an ion-exchange material of liquid, solid, and/or gas that can be coated. The coating can cover the entire exterior surface of the particle or substantial portions thereof. The coating can cover portions of the interior surfaces of the particle. The coating can be irreversible to permanently coat the particle, or reversible to release the particle upon dissolution of the coating. The particle can be a single material or an agglomerate of materials that can be prepared by, for example, fusion, sintering, pressing, compressing, phase separation, precipitation, aggregation and coalescence, or otherwise formed together. The particle can have any shape either regular or irregular such as spherical, elliptical, triangular, cylindrical, etc.

The term "material" as used herein refers to any substance on a molecular level or in bulk and can be a liquid and/or solid, e.g. an emulsion or a resin.

The term "pore size" as used herein refers to a mean measurement, providing a guideline that particles larger than the pore size are less likely to penetrate into the interior of the particle, while smaller particles are more likely to penetrate into the interior of the particle. It is to be understood that the particles admitted to or deflected from a pore are not necessarily exactly the "pore size" given. That is, admittance to or exclusion from the pore is based on many factors, including actual pore size (wherein each pore of a core can have a different size), steric hindrance factors, ionic attractions, polarizations, and the like. Additionally, some particles, such as microporous gel type ion exchange materials, do not have defined pores. The particles have a "pore size that is defined by the intermolecular spacing within the gel matrix to define the size exclusion limit.

The term "ion-exchange" as used herein refers to the process wherein each charge equivalent that can be "coupled" or "captured" on the ion-exchange surface can release an equivalent charge into an appropriate solution. This displacement of counter-ions from the ion-exchange core can release a large number of counter-ions into a sample solution. The selectivity of the ion-exchange core can be greater for the ion to be removed from the sample solution than for the counter-ion of the ion-exchange core. Ions of similar affinity as the counter-ion establish an equilibrium distribution based on the relative affinity of the ions for the ion-exchanger. The equilibrium can either provide or not provide the uptake of ions from solution. The counter-ion can be almost any ion including chloride, hydroxide, acetate, formate, bromide, sulfate, nitrate, phosphate or any other organic or inorganic anion. The choice of counter-ion can be influenced by the nature of the ions in solution that are to be removed. A counter-ion can be selected that has a significantly lower affinity for the ion-exchange core relative to the ion in solution, thus providing exchange with the ion in solution. Neutralization using a cation exchange resin in a mixed bed can drive the uptake of an ion from solution. This can be the case even if the affinity of the cation for the resin is lower than the affinity for the counter-ion. While the above describes the use of anion-exchange particles, the present teachings are analogous for cation-exchange particles. Counter-ions for cation-exchange particles include hydronium, sodium, potassium, ammonium, calcium, magnesium, or any other organic or inorganic cation. Polyelectrolyte-coated ion-exchange particles can be prepared in any ionic form.

The term "mixture" as used herein refers to more than one polyelectrolyte-coated particle used together in a packed column, a mixed-bed, a homogenous bed, a fluidized bed, a static column with continuous flow, or a batch mixture, for example. The mixture can include polyelectrolyte-coated cation-exchange particles, polyelectrolyte-coated anion-exchange particles, uncoated cation-exchange particles, uncoated anion-exchange particles, inerts, or any combination thereof. The mixture can include any physical configuration known in the art of separations, and any chemical mixture known in the art of ion exchange. The mixture can be any proportion including stoichiometric equivalent amounts. A mixture of particles can provide size-based removal with desalting of the solution. An example is a polyelectrolyte-coated ion-exchange particle in the hydroxide form in a mixed bed with cation-exchange particles in a hydronium form. A mixture of particles can provide size-based removal of small ions without desalting the solution. An example is a polyelectrolyte-coated ion-exchange particle in the chloride or acetate form (or any other anion other than hydroxide), and no cation exchange material. The choice of counter-ionic form used for the polyelectrolyte-coated ion-exchange particles can be based on the application for which they are to be implemented.

The term "coating" and grammatical variations thereof as used herein refer to less than a monolayer, a monolayer, or multiple layers of a polyelectrolyte with the same charge, or multiple layers of varied polyelectrolytes with opposite charges covering the particle. Smaller molecules, such as, for example, inorganic buffer ions, and nucleotides can penetrate or permeate through the coating and can be retained by or ion-exchanged with the particle. The coating can prevent larger molecules, such as, for example, nucleic acids, from penetrating or permeating through the coating and reacting with the particle.

The terms "polymer," "polymerization," "polymerize," "cross-linked product," "cross-linking," "cross-link," and other like terms as used herein are meant to include both polymerization products and methods, and cross-linked products and methods wherein the resultant product has a three-dimensional structure, as opposed to, for example, a linear polymer. The term "polymer" also refers to oligomers, homopolymers, and copolymers. Polymerization can be initiated thermally, photochemically, ionically, or by any other means known to those skilled in the art of polymer chemistry. According to various embodiments, the polymerization can be condensation (or step) polymerization, ring-opening polymerization, high energy electron-beam initiated polymerization, free-radical polymerization, including atomic-transfer radical addition (ATRA) polymerization, atomic-transfer radical polymerization (ATRP), reversible addition fragmentation chain transfer (RAFT) polymerization, or any other living free-radical polymerization.

The prefix "(meth)acryl" as used herein refers to methacryl and acryl. For example, N-methyl (meth)acrylamide refers to N-methyl methacrylamide and N-methyl acrylamide, and 2-hydroxyethyl (meth)acrylate refers to 2-hydroxyethyl methacrylate and 2-hydroxyethyl acrylate.

The term "DNA" as used herein refers to any nucleic acid, including RNA, PNA, and others as understood to one skilled in the art of molecular biology.

According to various embodiments, polyelectrolyte-coated particles can have many uses such as, for example, in the separation of biomolecules. According to various embodiments, polyelectrolyte-coated particles can provide separation of biomolecules by restricting the ability of large molecules to interact with ion-exchange active sites of the particle. Small molecules that can penetrate into the polyelectrolyte-coated particle can interact with the ion-exchange active sites and can be retained on those sites. Larger, highly charged species can be restricted from interacting with the ion-exchange core by the coating or by the pore size of the core particle. Such larger, highly charged species can remain in solution rather than bind to the ion-exchange particle. Larger species that remain in solution can be separated. Larger molecules are not immobilized on the coating. According to various embodiments, the small molecules can be eluted from polyelectrolyte-coated particles. According to various embodiments, large molecules can include single stranded DNA (ssDNA) fragments, and double stranded DNA (dsDNA) fragments, and small molecules can include nucleotides, short fragments of ssDNA, short fragments of dsDNA, and small ions such as chloride, acetate, and surfactants.

According to various embodiments, a polyelectrolyte-coated particle can be provided by exposing an ion-exchange core to an excess of polyelectrolyte. The core surface can become coated with the polyelectrolyte. The polyelectrolyte can be a biopolymer, including a naturally occurring biopolymer such as DNA, or a synthetic polymer as described herein.

According to various embodiments, a polyelectrolyte-coated particle can be provided by exposing an ion-exchange core to a polyelectrolyte containing charges opposite to that of the core. After the coating of the first polyelectrolyte, the coated particle is exposed to another polyelectrolyte containing charges opposite to that of the first polyelectrolyte. The coating process can be repeated to provide a polyelectrolyte-coated particle with multiple layers of alternative polyanion and polycation.

According to various embodiments, a coating including polyelectrolyte can decrease the interaction of large molecules, including ssDNA, with the core by a size sieving effect. The coating can cover the outer surface of an ion-exchange core, decreasing interaction of large molecules with the surface. The coating can create a size-exclusion barrier decreasing penetration of large molecules into the interior of the core particle. The chemical properties of the polyelectrolyte can determine the sieving properties that the polyelectrolyte-coated particle displays. Properties of the polyelectrolyte such as charge, charge density, hydrophobicity, tactility, flexibility, ratio of monomer units used in co- and ter-polymers, and molecular weight can all be modified in order to provide the desired sieving characteristics. According to various embodiments, the polyelectrolyte coating can be crosslinked in a later step to obtain desirable physical properties and size-exclusion characteristics.

According to various embodiments, a polyelectrolyte-coated particle can function as a size-excluded ion-exchanger by exploiting the inherent porosity of the ion-exchange core. Ion- exchange cores can be obtained with a wide variety of pore sizes, such as 5 angstroms (microporous) and 1000 angstroms or greater (macroporous). An ion-exchange core can be selected based on pore size such that it excludes molecules of a given size based on the requirements of the application. According to various embodiments, the polyelectrolyte coating can be large enough to be excluded from the pores of the ion-exchange core, thereby coating the exterior surface with substantially decreased coating of the interior of the pores. The polyelectrolyte coating can decrease the interaction of large molecules, such as ssDNA with the surface of the ion-exchange core by blocking a substantial amount of the surface ion-exchange sites. The pore size of the ion-exchange core bead can be small enough to decrease the penetration of large molecules, such as ssDNA, into the pores of the core and interacting with the core ion-exchange sites. According to various embodiments, the surface ion-exchange sites can be substantially blocked and the inner ion-exchange sites can become less accessible, such that the polyelectrolyte-coated particle retains significantly less large molecules, such as dsDNA. In contrast, smaller ions such as chloride, acetate, phosphate, pyrophosphate, small oligonucleotides, and nucleotides can enter the pores of the ion-exchange core and interact with interior ion-exchange sites. The coating can decrease the interaction of small ions, like the large molecules, with the surface ion-exchange sites because the surface sites are occupied by the polyelectrolyte coating. The resultant ion-exchange capacity of such a polyelectrolyte-coated particle (for small ions) is equal to the working capacity of the bare ion-exchange core minus the capacity of the surface of the core. The interior pores of the particle provide the substantial ion-exchange capacity of the polyelectrolyte-coated particle after the surface ion-exchange sites have been occupied by the polyelectrolyte coating.

According to various embodiments, a coating can be formed on an ion-exchange core such that the coating has a thickness of from less than an equivalent monolayer to multiple layers. The thickness of the coating can vary over the surface of the ion-exchange core, or the thickness of the coating can be uniform over the entire surface of the ion-exchange core. According to various embodiments, the coating can at least partially cover the ion-exchange core. The coating material can at least partially fill one or more pore or surface feature, for example, pores, cracks, crevices, pits, channels, holes, recesses, or grooves, of the ion-exchange core. For example, an ion-exchange core can be coated on all internal and external surfaces with a polyelectrolyte suitable for forming a coating.

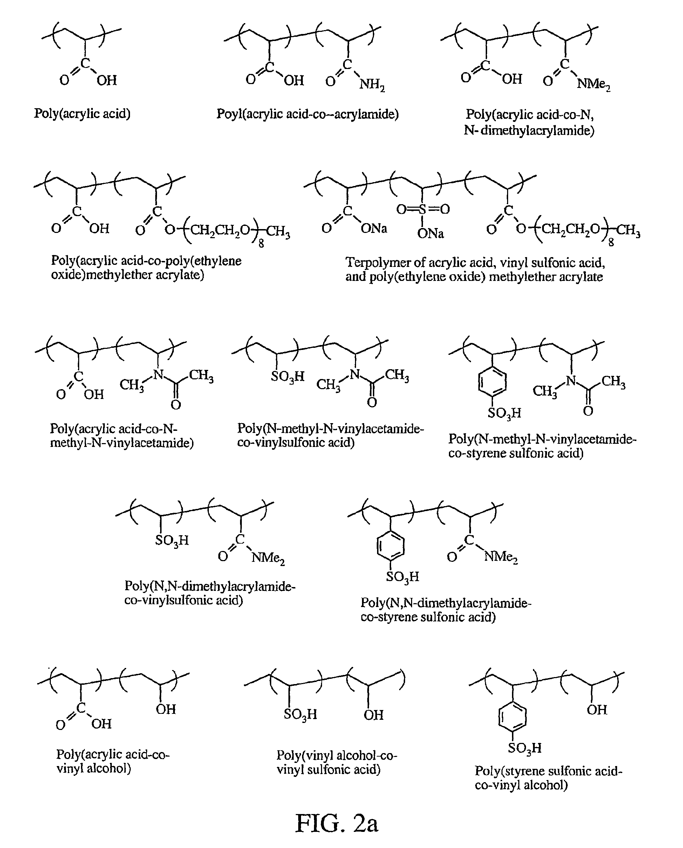

According to various embodiments, Fig. 1 illustrates polyelectrolyte-coated particle 30 which can include ion-exchange core 12 with pores 32 coated with a polyelectrolyte layer 20 composed of a biopolymer 300. Fig. 2 illustrates polyelectrolyte-coated particle 30 which can include ion-exchange core 12 with pores 32 coated with a polyelectrolyte layer 20 composed of a synthetic polymer 310. Small ionic particles (not shown) can sieve and/or enter pores 32 to bind to ion-exchange sites illustrated by positive charges, as in the case of anion-exchange core. The polyelectrolyte coating can substantially decrease the amount of large molecules illustrated by large ssDNA fragments that bind to the ion-exchange core.

According to various embodiments, the ion-exchange core can be an anionic or cationic material. The ion-exchange core can be a polymer, cross-linked polymer, or inorganic material, for example, silica. The ion-exchange core can be a solid core material capable of ion-exchange, or a solid core material treated with an ion-exchange resin. The ion-exchange core can be surface-activated. The ion-exchange core can be non-magnetic, paramagnetic, or magnetic. Exemplary ion-exchange core materials include those listed below.

According to various embodiments, ion-exchange material for the core can include anion-exchange resins such as Macro-Prep High Q, Macro-Prep 25Q, Aminex A-27, AG 1-X2, AG 1-X4, AG 1-X8, and AG 2-X8 (Bio-Rad, Hercules, CA, USA), Chromalite 30 SBG (Purolite Company, Bala Cynwyd, PA, USA), POROS HQ 20 (Applied Biosystems, Framingham, MA, USA), CA08Y and CA08S (Mitsubishi Chemical America, White Plains, NY, USA), Powdex PAO (Graver Technologies, Glasgow, DE, USA), Nucleosil SB (Alltech Associates, Inc., Deerfield, IL, USA), Fractogel TMAE (EM Science, Gibbstown, NJ, USA), IE 1-X8 (Spectrum Chromatography, Houston, TX, USA), Super Q-650S (TosoHaas Bioscience, Montgomeryville, PA, USA), TMAHP-100 (Iontosorb AV, Czech Republic), Chromalite 30 SBA (Purolite International Ltd., UK), and ANEX-QS (Transgenomic, Inc., San Jose, CA, USA). According to various embodiments, the cores material can include PMMA, PS-DVB, silica, and/or cellulose. According to various embodiments, ion-exchange cores can include cation-exchange resins provided by manufacturers similar to those for anion-exchange resins including Chromalite 30 SAG (Purolite International Ltd., UK), AG 50WX8 and Macro-Prep High S (Bio-Rad, Hercules, CA, USA). Other cation and anion resins that can be used as ion-exchange cores will be apparent to one of ordinary skill in the art of ion-exchange resins.

According to various embodiments wherein the ion-exchange core includes a solid core material capable of ion-exchange, the solid core material can be macroporous silica, controlled pore glass (CPG), a macroporous polymer microsphere with internal pores, other porous materials as known to those of ordinary skill in the art of ion-exchange separation, or a combination thereof. The solid core material can have various surface features, including, for example, pores, cracks, crevices, pits, channels, holes, recesses, or grooves. The solid core material can include sodium oxide, silicon dioxide, sodium borate, or a combination thereof. The solid core material can be surface-activated to be capable of ion-exchange, for example, modification to be capable of cation-exchange or anion-exchange. Modification of the solid core material can include treatment of the solid core material to form cationic or anionic substituent groups on the surfaces of the solid core material. As used herein, the term "surface" can include external surfaces and/or internal surfaces. Internal surfaces can be, for example, the surfaces of voids or pores within the solid core material. The solid core material can be surface-activated to include one or more of quaternized functional groups, carboxylic acid groups, sulfonic acid groups, other cationic or anionic functional groups known to those of ordinary skill in the art of ion-exchange separation, or a combination thereof, on the surface of the solid core material.

According to various embodiments, the biopolymer polyelectrolyte can be a naturally-occurring biopolymer such as DNA. Examples of naturally-occurring DNA include sheared salmon sperm DNA, plasmid DNA, restriction digests of plasmid DNA, herring sperm DNA, calf thymus DNA, and other naturally derived DNA. An example of commercially purchased DNA is sheared salmon sperm DNA (Eppendorf AG, Hamburg, Germany). According to various embodiments, the naturally-occurring biopolymer DNA that can be used as a polyelectrolyte in the coating is distinguished as non-sample DNA to indicate that its source is not the sample that has been subjected to the biological reaction.

According to various embodiments, an ion-exchange core can be coated with a synthetic-polymer polyelectrolyte. According to various embodiments, the ion-exchange core can be coated with a water-soluble, or at least slightly water-soluble, polyanion. According to various embodiments, polyanion containing anionic functional groups can be used for coating. The anionic functional group can include carboxylic, boric, sulfonic, sulfinic, phosphoric, or phosphorus group, or a combination thereof. Polyanions containing inorganic acid functional groups can also be used. According to various embodiments, the water-soluble, or at least slightly water-soluble, polyanion can be prepared by copolymerization of an acid- or phenolic-containing monomer, for example, acrylic acid, methacrylic acid, 4-acetoxystyrene that can be hydrolyzed to give phenolic group, 4-styrenesulfonic acid, styrylacetic acid, or maleic anhydride, with a water soluble, at least slightly water-soluble or water-insoluble, co-monomer. According to various embodiments, the synthetic polymer can be can be a homopolymer, a copolymer, a terpolymer, or another polymer.

According to various embodiments, the synthetic polymer can include monomers including: (meth)acrylamide, N-methyl (methyl)acrylamide, N,N-dimethyl (methyl)acrylamide, N-ethyl (meth)acrylamide, N-n-propyl (meth)acrylamide, N-isopropyl (meth)acrylamide, N-ethyl-N-methyl (meth)acrylamide, N,N-diethyl (meth)acrylamide, N-hydroxymethyl (meth)acrylamide, N-(3-hydroxypropyl) (methy)acrylamide, N-vinylformamide, N-vinylacetamide, N-methyl-N-vinylacetamide, vinyl acetate that can be hydrolyzed to give vinylalcohol after polymerization, 2-hydroxyethyl (meth)acrylate, 3-hydroxypropyl (meth)acrylate, N-vinypyrrolidone, poly(ethylene oxide) (methy)acrylate, N-(meth)acryloxysuccinimide, polyanethole sulfonate N-(meth)acryloylmorpholine, N-2,2,2-trifluoroethyl (meth)acrylamide, N-acetyl (meth)acrylamide, N-amido(meth)acrylamide, N-acetamido (meth)acrylamide, N-tris(hydroxymethyl)methyl (meth)acrylamide, N-(methyl)acryloyltris(hydroxymethyl)methylamide, (methyl) acryloylurea, vinyloxazolidone, vinylmethyloxazolidone, or a combination thereof. According to various embodiments, the synthetic-polymer polyelectrolyte can be poly(acrylic acid-coN,N-dimethylacrylamide) or poly(N,N-dimethyl acrylamide-co-styrene sulfonic acid).

According to various embodiments, the ion-exchange can be coated with a water-soluble, or at least slightly water-soluble, polycation. According to various embodiments, polycation containing cationic functional groups can be used for coating. The cationic functional group can include protonated primary, secondary, and tertiary amine. According to various embodiments, the water-soluble, or at least slightly water-soluble, polycation can be prepared by copolymerization of a positively charged monomer with a water-soluble, at least slightly water-soluble, or water-insoluble comomer. Examples of polycation can include allyl amine hydrochloride, (3-acrylamidopropyl)trismethylammonium chloride, N-(3-aminopropyl)methacrylamide hydrochloride, and N-vinyl amides that can be hydrolyzed to give an amino group. According to various embodiments, the synthetic polymer can be poly(N-(3-aminopropyl)methacrylamide-co-N,N-dimethylacrylamide).

According to various embodiments, Fig. 2a illustrates the monomeric subunits for those synthetic polymers listed. The abbreviations include acrylic acid (AA), acrylamide (AAm), N.N-dimethylacrylamide (DMA), (polyethylene oxide)monoacrylate (PEOacrylate), and vinyl sulfonic acid (VSA). According to various embodiments, the preparation of synthetic polymer can provide weight average molecular weights (M_{w}) ranging from 50 kiloDaltons to 15.0 megaDaltons, or 200 kiloDaltons to 4.0 megaDaltons, or 500 kiloDaltons to 3.0 megaDaltons. According to various embodiments, the molar percentage of the negatively or positively charged comonomer can contribute from 0.01 percent to 100 percent, or 0.1 percent to 20.0 percent, or 1.0 percent to 10.0 percent.

According to various embodiments, other synthetic polymers can include homopolymers of styrene sulfonic acid, homopolymers and copolymers of acrylic acid, methacrylic acid, vinyl sulfonic acid, styrene sulfonic acid, 4-acetoxystyrene (precursor of 4-hydroxystyrene), and vinylphosphonic acid. According to various embodiments, other synthetic polymers can include homopolymers and copolymers of allyl amine hydrochloride, (3-acrylamidopropyl)trimethylammonium chloride, N-(3-aminopropyl)methacrylamide hydrochloride. The comonomers can include acrylamide, methacrylamide, vinyl acetate that can be converted into vinyl alcohol in a subsequent step, *N,N-*dimethylacrylamide, *N*-ethylacrylamide, *N*-propylacrylamide, *N*-vinyl-*N-*methyl acetamide, 2-hydroxyethyl acrylate, and vinyl methyl ether.

According to various embodiments, the polyelectrolyte can include polyanions such as poly(styrenephosphoric acid), poly(phosphoric acid), homo-polymers and co-polymers of maleic acid, derivatives thereof and the like, homo-polymers and co-polymers of fumaric acid, derivatives thereof and the like, peptide-type synthetic polyanions such as poly(aspartic acid), poly(galactronic acid), poly(glutamic acid), nucleic acid type synthetic polyanions such as poly(adenylic acid), poly(inosinic acid), poly(uridylic acid), and natural polyanions such as polysaccharides.

According to various embodiments, the ion-exchange core can be coated with multiple layers of polyelectrolyte. The polyelectrolyte layers can include alternating layers of polyanions and polycations. Such alternating layers can provide strength durability and means to tailor the permeability of the coating. Provided the outer most polyelectrolyte of the multiple-layer structure is negatively charged, DNA fragments will not be immobilized from its solution. Small anions such as, for example, chloride and primers can penetrate or permeate through the multiple-layer structure to be coupled to the core.

According to various embodiments, the polyelectrolyte-coated particles can be provided as a mixture. The mixture can be incorporated in bed or column. According to various embodiments, the polyelectrolyte-coated particles can be provided in a bulk mode. A well-formed chromatographic bed of polyelectrolyte-coated particles is not necessarily required.

According to various embodiments, the polyelectrolyte-coated particles can be provided in a device. The device can be a microfluidic device having one or more pathway, wherein at least a portion of at least one pathway includes polyelectrolyte-coated particles. The device can have an inlet and an outlet in fluid communication with the polyelectrolyte-coated particles. The particles can be present in, for example, a column. As used herein, a column can be in a horizontal or vertical orientation, or in any position between a horizontal and a vertical orientation. The column can include a receptacle such as a cavity, chamber, reservoir, well, reaction region, bed, recess, or other receptacle suitable for containing or retaining polyelectrolyte-coated particles and the reaction products. The column can contain a plurality of polyelectrolyte-coated particles. The outlet of the device can be in fluid communication with a receptacle, such as a purified sample well, a tube, a glass plate, or another means of collecting a purified sample. According to various embodiments, the plurality of polyelectrolyte-coated particles can be a mixture.

According to various embodiments, a device for separating the reaction products can be provided. The device can include a mixture of polyelectrolyte-coated particles. The method can include adding the particles to the column of the device. The reaction products can be placed in or introduced to the inlet of the device. The reaction products can travel from the inlet through the column including polyelectrolyte-coated particles and optional additional material. The sample can be subjected to a combination of size-exclusion separation and ion-exchange resulting in a filtration and/or purification of the reaction products. The filtered and/or purified solution can be eluted or removed from the column through the outlet, and can be directed to a receptacle for analysis and/or further processing. The reaction products can be moved through the column by centripetal force. According to various embodiments, the plurality of polyelectrolyte-coated particles can be mixed with reaction products in bulk. The plurality of polyelectrolyte-coated particles form a first volume and the reaction products form a second volume where the first volume is less than or equal to the second volume.

According to various embodiments, separation of the reaction products using polyelectrolyte-coated particles can be achieved using a volume of polyelectrolyte-coated particles that is sufficient to provide adequate ion-exchange capacity, such as, ion-exchange of at least 80%, at least 90%, or at least 95% of the reaction products. The separation can occur in ten minutes or less, five minutes or less, or two minutes or less. The separation can include contacting the reaction products with the polyelectrolyte-coated particles for a period of time sufficient for the polyelectrolyte-coated particles to ion-exchange with the reaction products, and removing the purified reaction products from the polyelectrolyte-coated particles.

According to various embodiments, separating the purified reaction products from the particles can include removing the purified reaction products from the polyelectrolyte-coated particles, removing the particles from the purified reaction products, and/or sampling the purified reaction products from the mixture of particles and purified reaction products. An example of sampling the purified reaction products from the mixture of particles and purified reaction products can include analyzing the product in a tube by dipping a capillary directly into the tube and injecting into an instrument for further analysis.

According to various embodiments, separations for the polyelectrolyte-coated particles can include, for example, separation of polymerase chain reaction (PCR) products, separation of DNA sequencing reaction mixtures, and purification of RNA. Polyelectrolyte-coated particles can also be used for purification and/or separation of, for example, oligonucleotides, ligase chain reaction products, proteins, antibody binding reaction products, oligonucleotide ligation assay products, hybridization products, and antibodies. Polyelectrolyte-coated particles can also be used for desalting of biological products or reaction mixtures.

According to various embodiments, the selectivity of the polyelectrolyte-coated particles can depend on at least one of these criteria: (1) the molecular weight of the polyelectrolyte in the coating, (2) the chemical nature of the charged functionality and the charge density or molar percent of the charge of the polyelectrolyte in the coating, (3) the pore size of the ion-exchange core, (4) the nature of co-monomer in the coating polymer, and (5) the ratio of various co-monomers in the polymer. Each separation can provide different desirable features for at least one of the above criteria.

According to various embodiments, PCR products include materials that can interfere with downstream analysis. PCR products can include amplified target sequences (amplicons), buffer salts, surfactants, metal ions, enzymes (e.g. polymerase), nucleotides, oligonucleotide primers, and other components in solution. According to various embodiments, the target sequences of double-stranded DNA (dsDNA) can be analyzed, or used in subsequent enzymatic reactions that can be sensitive to at least some of the other PCR products. For example, free nucleotides and oligonucleotide primers can interfere with downstream enzymatic reactions. According to various embodiments, a polyelectrolyte-coated particle can separate nucleotides, oligonucleotide primers, and buffer salts from the target sequences of dsDNA. The resulting solution can contain purified PCR products in a desalted environment, and can be used in downstream reactions and analyses.

According to various embodiments, PCR purification can be directed toward separating larger double stranded DNA (dsDNA) from smaller ssDNA, and dsDNA (e.g. primer-dimer, an unwanted side reaction product which is a dsDNA, or other non-specifically amplified fragments), free nucleotides, and salts. According to various embodiments, PCR products for removal include nucleotides, primers with 45 nucleotides of ssDNA, primer-dimer with 60 bp of dsDNA, and dsDNA fragments smaller than 200 bp. According to various embodiments, primers, primer-dimer, and DNA fragments smaller than 100 bp can be captured by polyelectrolyte-coated particles. According to various embodiments, primers, primer-dimer, and DNA fragments smaller than 300 bp can be captured by polyelectrolyte-coated particles.

According to various embodiments, separation of larger dsDNA from other PCR products can include desalting. According to various embodiments, separation of larger dsDNA from other PCR products does not include desalting. There are circumstances when desalting can interfere with downstream processing such as separation and detection of the larger dsDNA PCR products.

According to various embodiments, the polyelectrolyte-coated particles can be subjected to the same PCR conditions as the PCR reactants prior to PCR termination and purification. The polyelectrolyte-coated particles can be subjected to temperature cycling from 65 to 95 °C. The polyelectrolyte-coated particles can be bundled into a device that provides PCR and subsequent purification.

According to various embodiments, an ion-exchange core with a pore size in the range of 100 Angstroms to 2000 Angstroms, coated with a polyelectrolyte of M_{w} in the range of 1.0 megaDaltons to 3.0 megaDaltons provides PCR purification. According to various embodiments, an ion-exchange core with a pore size of 1000 Angstroms, coated with a polyelectrolyte of M_{w} of 1.7 megaDaltons to 2.6 megaDaltons provides PCR purification.

According to various embodiments, DNA sequencing reaction products can include material that can interfere with downstream analysis. The quality of separation for purification sequencing reaction products can be evaluated by analyzing at least one of these criteria: (1) residual dye artifacts ("blobs") that appear as broad peaks superimposed over the sequence data, (2) peak intensity balance between large or small fragments, and (3) desalting of the sequencing reaction products.

According to various embodiments, DNA sequencing reaction products can include dye-labeled target sequences (the "sequencing ladder"), buffer salts, phosphate and pyrophosphate ions, metal ions, enzymes (e.g. polymerases), nucleotides, oligonucleotide primers, dye-labeled oligonucleotide primers, and other components such as residual, unincorporated dye-labeled-dideoxynucleotides ("dye terminators"). According to various embodiments, the dye-labeled target sequences can be subjected to electrophoretic analysis and DNA sequencing ("basecalling") that can be sensitive to at least some of the other sequencing reaction products resulting in "blobs" that can cause errors in "basecalling." According to various embodiments, capillary sequencers can use electrokinetic injection to introduce sequencing reaction products into the capillary for electrophoretic separation. The presence of salts in the sequencing reaction products can affect their introduction into the capillary, where a reduced salt concentration can enhance injection into the capillary.

According to various embodiments, polyelectrolyte-coated particles can remove material that produce "blobs" from and desalt sequencing reaction products. According to various embodiments, sequencing reaction products purified with polyelectrolyte-coated particles can have a salt concentration of less than or equal to 100 µM or less than or equal to 50 µM. A sample solution purified by polyelectrolyte-coated particles can be suitable for electrokinetic capillary injection. For these and other purposes, the polyelectrolyte-coated particle can have a size-exclusion limit of, for example, less than 10 nucleotides ssDNA, and can be able to remove small ions such as salts and dye-labeled primers from a sample solution while leaving ssDNA free in solution. Sequencing reaction purification using polyelectrolyte-coated particles can be used to separate ssDNA, for example, having a size of from 10 nucleotides to 1500 nucleotides or larger in size, from smaller components such as, for example, dye-labeled primers and salts.

According to various embodiments, separation of DNA sequencing reaction products can include separating primers, dye-labeled primers, and salts from dye-labeled ssDNA targets by substantially excluding dye-labeled ssDNA fragments having greater than 45 nucleotides.

According to various embodiments, purification of a sequencing reaction sample can remove dye-labeled dideoxynucleotides and salts from the sequencing reaction products by allowing such components to pass through the coating and react with the ion-exchange core, leaving a purified sample containing an amount of ssDNA relative to the pre-filtered amount, in an amount of 70% or more, 80% or more, 90% or more, or 95% or more.

According to various embodiments, an ion-exchange core with a pore size in the range of 5 Angstroms to 1000 Angstroms, coated with a polyelectrolyte of M_{w} in the range of 1000 Daltons to 6.0 megaDaltons provides sequencing reaction purification. According to various embodiments, an ion-exchange core with a pore size of 10 Angstroms to 50 Angstroms, coated with a polyelectrolyte of M_{w} of 2.4 megaDaltons to 4.9 megaDaltons provides sequencing reaction purification.

According to various embodiments, a method for purifying DNA sequencing reaction products can include providing a plurality of polyelectrolyte-coated particles, and contacting the DNA sequencing reaction products to separate dye-labeled ssDNA fragments. The method can include removing residual dye artifacts such as dye-labeled primers that can result in blobs in the sequencing analysis. The method can include maintaining dye-labeled ssDNA fragment lengths.

### EXAMPLES

### COATING PARTICLES WITH BIOPOLYMER:

The ion-exchange resin was converted to an ion-exchange by washing a 1OOuL volume of resin with 1000uL of 1M salt, acid, or base solution. The mixture was vortexed for 5 minutes, and spun down in a centrifuge. The supernatant was removed and another 1000uL aliquot of salt, acid, or base solution was added. This was repeated three times. The ion-exchange core was then washed and spun five times using 1000uL aliquots of DI water.

The ion-exchange cores were coated with DNA by repeated washings with 100uL aliquots of 1mg/mL sheared salmon sperm DNA (Eppendorf AG, Hamburg, Germany). Coating was performed by washing a 100uL volume of resin with 100uL of 1mg/mL sheared salmon sperm DNA. The mixture was vortexed for 5 minutes, and spun down in a centrifuge. The supernatant was removed and another 100uL aliquot of 1mg/mL sheared salmon sperm DNA was added. This was repeated two times. The SEIE particles were then washed and spun three times using 1000uL aliquots of DI water.

### COATING PARTICLES WITH SYNTHETIC POLYMER:

Poly(AA-co-DMA) polyelectrolyte for coating particles was provided by free radical polymerization of 0.32 g (4.44 mmol) of acrylic acid with 8.04 g (81.07 mmol) of N,N-dimethylacrylamide in 200 mL of DI water at 45 °C for 15 hours, using ammonium persulfate as an initiator and N,N,N'N'-tetramethylethylenediamine as a catalyst. The resulting polymer was purified by dialysis (50K MWCO) and lyophilization to provide 7.70 g (92 % yield) of the polymer; M_{w} = 3.40 MDa, Mn = 2.67 MDa.

Prior to coating, the anion-exchange resin was first converted into hydroxide anion-exchange core in the same manner as the previous example. A 1000 µL aliquot of DI water was added to 0.20-0.25 mL volume of an anion-exchange core in chloride form. The mixture was vortexed for 2 minutes, and spun down in a centrifuge. The supernatant was removed and this DI water washing was repeated two times. A 1000 µL aliquot of 2.0 M of ammonium hydroxide was added to the washed resin. The mixture was vortexed for 2 minutes, let standing for 5 minutes at ambient temperature, vortexed one minute, and spun down in a centrifuge. The supernatant was removed and this ammonium hydroxide washing was repeated two times. A 1000µL aliquot of DI water was added to the pellet of ion exchange particles, vortexed for 1 minute, spun down by a centrifuge, and supernatant removed. This final DI water washing was repeated one more time. The resin was re-dispersed in 500 µL of DI water in a snap-capped polypropylene micro-centrifuge tube and stored in a refrigerator prior to use.

To a 1.5 mL microcentrifuge tube containing 15-20 µL of the wet anion exchange resin, 1.0 mL of the polymer solution (0.5 weight percent solution in deionized water) was added. It was vortexed for 1 minute, let standing at ambient temperature for 5 minutes, vortexed for additional one minute, spun down in a centrifuge, and supernatant removed. The polymer solution coating was repeated one more time. The pellet was then washed with 1 mL of DI water and spun down three times. The final washed resin was re-dispersed in 500 µL of DI water and stored in a refrigerator prior to use.

Alternatively, poly(AA-co-DMA) polyelectrolyte for coating particles was provided by polymerization under inert atmosphere (ultra pure nitrogen). To a 500-mL round bottom three-neck flask equipped with a 2" Teflon stirring blade, a glass bleeding tube for purging, and a water-cool condenser, was charged with 150.0 mL of Milli-Q water, 8.0160 g (80.86 mmol) of re-distilled *N*,*N*-dimethylacrylamide (Dajac), 0.3285 g (4.56 mmol) of redistilled acrylic acid (Aldrich Chemical) and 0.8043 g of an 1.9972 wt% aqueous solution of ammonium persulfate. This mixture was purged with ultra pure nitrogen at 150 mL/min for 60 minutes while stirred at a constant speed of 200 rpm. To purged solution, 80 µL of *N*,*N*,*N*'*N*'-tetramethylethylenediamine (Electrophoresis reagent from Aldrich Chemical) was added. The mixture was lowered into an oil bath at 50±1 °C and stirred at 200 rpm for a period of 3.5 hours. At the end of the reaction time, 50 mL of DI water was added and stirred for 5 minutes. The resulting water-clear solution was dialyzed with 50K MWCO Spectra/Pro membrane in 5 Gal of DI water for 4 days, with water changed twice every 24 hours. The dialyzed solution was lyophilized to give 7.70 g (92.0 % yield) of copolymer. Molecular weight was determined by GPC/MALLS to be 2.4 MDa Mw and 1.26 MDa Mn.

### DNA SEQUENCING REACTION PURIFICATION BY POLYELECTROLYTE-COATED PARTICLES WITH BIOPOLYMER:

For DNA sequencing reaction purification, a sample was prepared containing 400 uL dRhodamine Terminator Ready Reaction Mix (Applied Biosystems, Foster City, CA, USA), 50 uL M13 universal reverse primer (3.2 pmol/uL), 25 uL template-amplicon (~100 ng/uL), and 525 uL DI water. This solution was aliquoted into wells in a thermal cycler plate at a volume of 20uL/well. The mixture was subjected to 25 cycles of heating, wherein each cycle included heating at 95°C for 10 seconds, heating at 50°C for 5 seconds, and heating at 60°C for 120 seconds.

To provide the polyelectrolyte-coated particles with a biopolymer, 100uL each of Aminex A-27, Bio-Rad AG 1-X8, and Bio-Rad AG 2-X8 were prepared as hydroxide polyelectrolyte-coated particles as described above. The resins were coated with 1mg/mL sheared salmon sperm DNA as described above and washed with DI water. 100uL of each of the coated anion exchange particles was mixed with 100uL of Chromalite 30 SAG, prepared as hydrogen-form polyelectrolyte-coated particles, as described above, to form a mixed ion exchange bed. The mixed beds were washed with 1mg/mL sheared salmon sperm DNA as described above and washed with DI water. 2uL of the each of the coated mixed beds were added to a small MicroAmp® tube (Applied Biosystems, Foster City, CA, USA). A mixed bed prepared from uncoated Aminex A-27 and Chromalite 30 SAG was prepared as a control.

To perform the purification, 1uL of the above described sequencing reaction was added to each MicroAmp tube containing 2uL of the DNA-coated mixed bed ion exchange resins. The tubes were vortexed for 5 minutes after which 5uL DI water was added to each tube and mixed with a pipette. The microcentrifuge tubes were spun at 5000x g and 5 µL of supernatant was removed from each tube and pipetted into a 96 well plate for analysis on a ABI Prism® 3100 sequencer. The samples were analyzed on the ABI Prism® 3100 using a 36 cm array, POP6® polymer (Applied Biosystems), and a standard sequencing module.

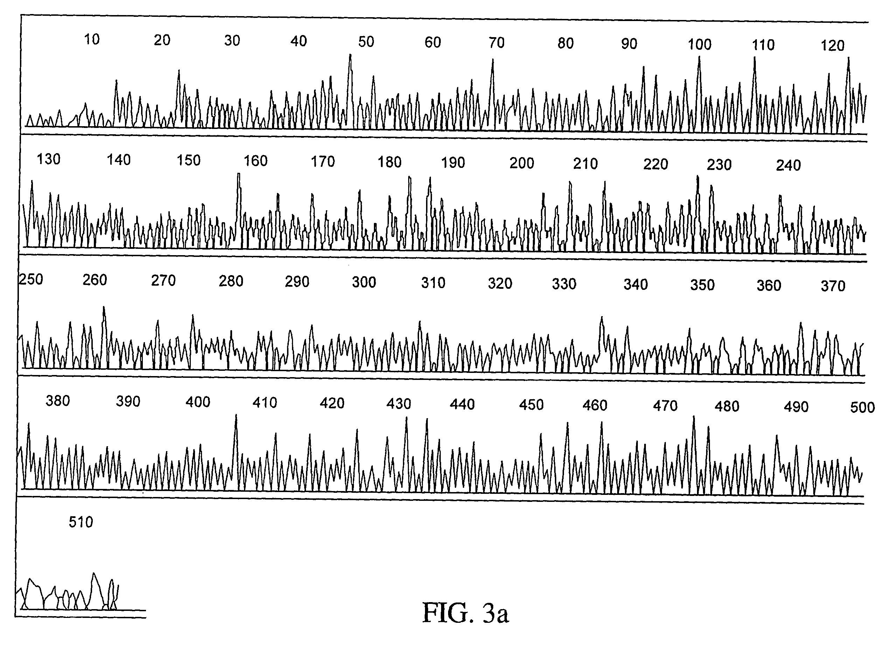

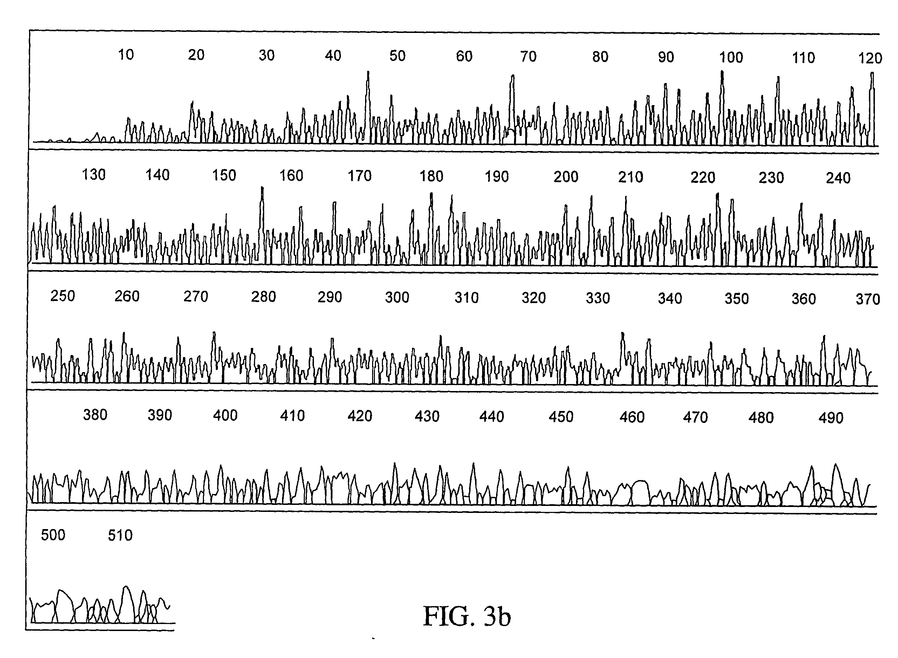

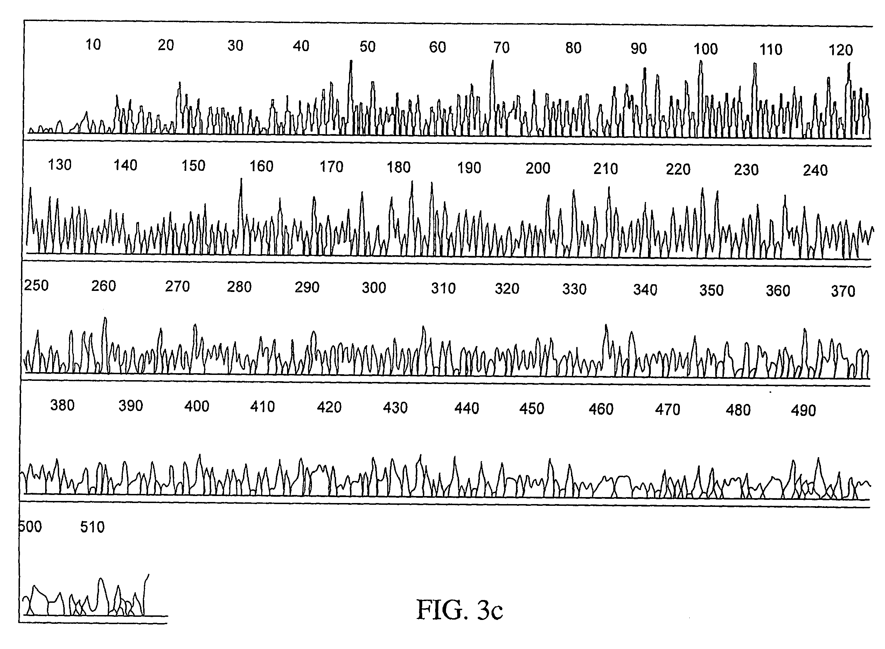

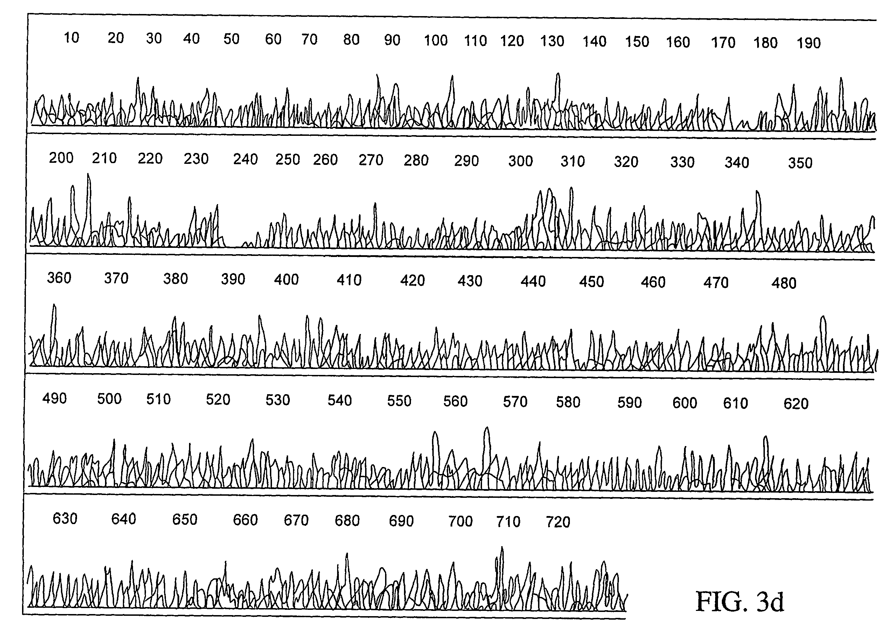

Figs. 3a-3d illustrate the results from this purification. Figs. 3a-3c illustrate that the biopolymer-polyelectrolyte-coated particles provided desirable purification (Fig. 3a illustrating DNA-coated Aminex A-27, Fig. 3b illustrating DNA-coated Bio-Rad AG 1-X8, and Fig. 3c illustrating DNA-coated Bio-Rad AG 2-X8, all three in a cationic-anionic mixed bed). For example, the substantial removal of dye blob (residual dye-labeled ddNTPs) and the relatively high signal strength indicated a desirable desalting of the sample. By contrast, Fig. 3d illustrates that the uncoated mixed bed (Aminex A-27 mixed with Chromalite 30 SAG) did not provide desirable purification. Loss of most of the DNA fragments was observed as is expected from purification with an uncoated anion exchange resin.

### PCR REACTION PURIFICATION BY POLYELECTROLYTE-COATED PARTICLES WITH BIOPOLYMER:

To demonstrate PCR reaction purification, a sample was prepared using a fluorescently-labeled primer so that the PCR product could be analyzed on a fluorescent capillary sequencer. A solution was prepared containing 102 uL PCR master mix (Applied Biosystems), 4 uL FAM-labeled forward primer (20 pmol/uL), 4 uL reverse primer (20 pmol/uL), 20 uL human gDNA, CEPH 1347-02 (50 ug/uL), and 70 uL deionized water (DI). This solution was aliquoted into wells in a thermal cycler plate at a volume of 20uL/well. The mixtures were heated at 96°C for 5 minutes, followed by 40 cycles of heating, wherein each cycle included heating at 96°C for 30 seconds, then at 60°C for 120 seconds.

Particles coated with a biopolymer, similar to the previous example were provided. To perform the purification, 1uL of the above described solution was added to a MicroAmp tube containing 2uL of the DNA-coated mixed bed ion exchange resins. The tube was vortexed for 5 minutes after which 5uL DI water was added to the tube and mixed with a pipette. The microcentrifuge tube was spun at 5000x g and 5 µL of supernatant was removed, added to 5uL deionized formamide, and pipetted into a 96 well plate for analysis on a ABI Prism® 3100 sequencer. The samples were analyzed on the ABI Prism® 3100 using a 36 cm array, POP-6® capillary electrophoresis polymer (Applied Biosystems), and a standard fragment analysis module.

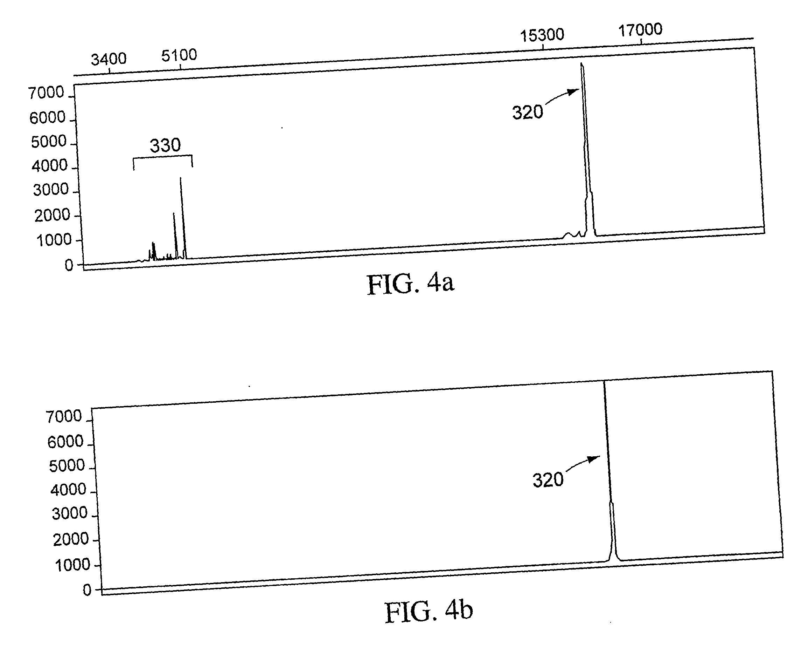

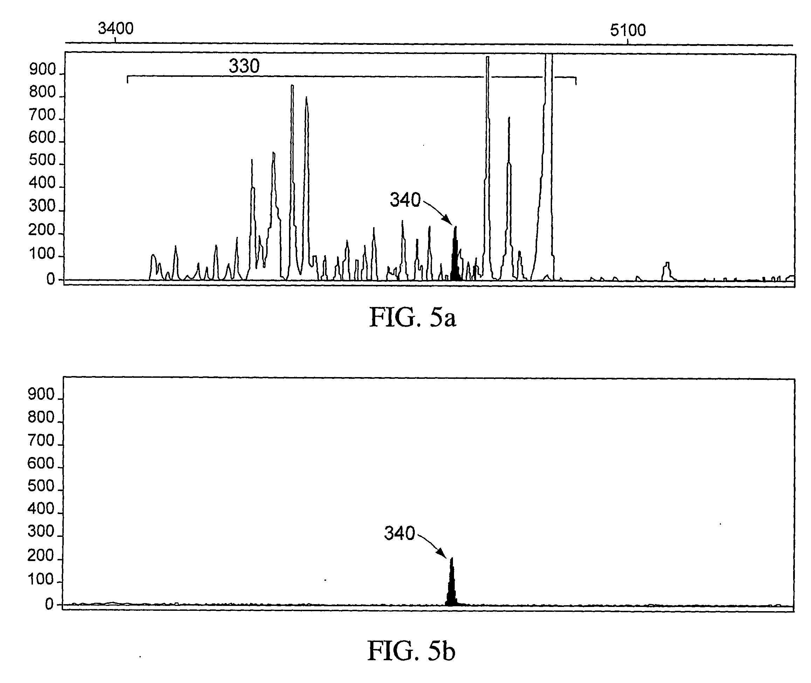

Figs. 4 and 5 illustrate the results from this purification as analyzed using GeneScan® software. The experiment was designed to observe the removal of the majority of a dye-labeled primer from a 550bp PCR product while retaining a double stranded DNA amplicon. Fig. 4a illustrates the PCR reaction solution prior to purification. Fig. 4b illustrates the PCR reaction solution purified and desalted by polyelectrolyte-coated particles. Fig. 4a illustrates peaks from 4000-5000 scans, labeled 330, generated by the primer, while the peak at 15,500 scans, labeled 320, was generated by the 550bp amplicon. The presence of PCR primer can often interfere with subsequent analyses of the PCR product or with subsequent reactions. Removal of the primer from PCR product can be desirable. Fig. 4b illustrates the resulting data when the PCR product is purified as described in the previous paragraph using polyelectrolyte-coated particles with biopolymer. The 550bp amplicon, labeled 320, is still visible, but the primer has been reduced to a less than detectable amount.

Figs. 5a and 5b are a magnified view of a region of the electrophorograms illustrated in Figs. 4a and 4b, focusing on the region of the PCR primer, labeled 330. An internal standard was added to the injection solution after the purification step, but prior to analysis on the ABI Prism® 3100. The internal standard, a synthetic ROX-labeled oligonucleotide at 20nM concentration, was used to measure the relative injection efficiency from the two solutions. The internal standard was observed in both electrophorograms, labeled 340. Fig. 5 illustrates that the injection efficiency from the two solutions is similar. Using the internal standard to normalize the peak heights, the reduction in primer is 100%, while the loss of PCR product is 22%. This example illustrates that contact with the polyelectrolyte-coated particles can substantially decrease the primer from the PCR solution while leaving 78% of the PCR product in solution. Contact of the same solution with uncoated ion exchange beads resulted in loss of most of the primer as well as all of the PCR product (not shown).

### DNA SEQUENCING REACTION PURIFICATION BY POLYELECTROLYTE-COATED PARTICLES WITH SYNTHETIC POLYMER:

For DNA sequencing reaction purification, a sample was prepared using a fluorescently-labeled primer so that the sequencing reaction product could be analyzed on a fluorescent capillary sequencer. A solution was prepared containing 400 uL dRhodamine Terminator Ready Reaction Mix (Applied Biosystems, Foster City, California, 50 uL M13 universal reverse primer (3.2 pmol/uL), 25 uL template-amplicon (~100 ug/uL), and 525 uL DI water. This master solution was aliquoted into wells in a thermal cycler plate at a volume of 20uL/well. The mixture was subjected to 25 cycles of heating, wherein each cycle included heating at 95°C for 10 seconds, heating at 50°C for 5 seconds, and heating at 60°C for 120 seconds.

To provide the polyelectrolyte-coated particles coated with a synthetic polymer, 20uL of Bio-Rad Macro-Prep High Q ion exchange resin was prepared as hydroxide polyelectrolyte-coated particles as described herein. The resin was coated with poly(acrylic acid-co-N,N-dimethylacrylamide), hereafter referred to as poly(AA-co-DMA), as described herein and washed with DI water. 20uL of poly(AA-co-DMA)-coated Macro-Prep High Q was mixed with 20uL of Chromalite 30 SAG (as hydrogen polyelectrolyte-coated particles, as described herein). 5uL of the ion exchange particle mixture was added to a small MicroAmp® tube (Applied Biosystems, Foster City, CA, USA).

To provide purification, 1uL of the above described sequencing reaction was added to a MicroAmp tube containing 5uL of the DNA-coated mixed bed ion exchange resins. The tube was vortexed for 5 minutes after which 5uL DI water was added to the tube and mixed with a pipette. The microcentrifuge tube was spun at 5000x g and 5 µL of supernatant was removed and pipetted into a 96 well plate for analysis on a ABI Prism® 3100 sequencer. The samples were analyzed on the ABI 3100 using a 36 cm array, POP-6® (Applied Biosystems), and a standard sequencing module.

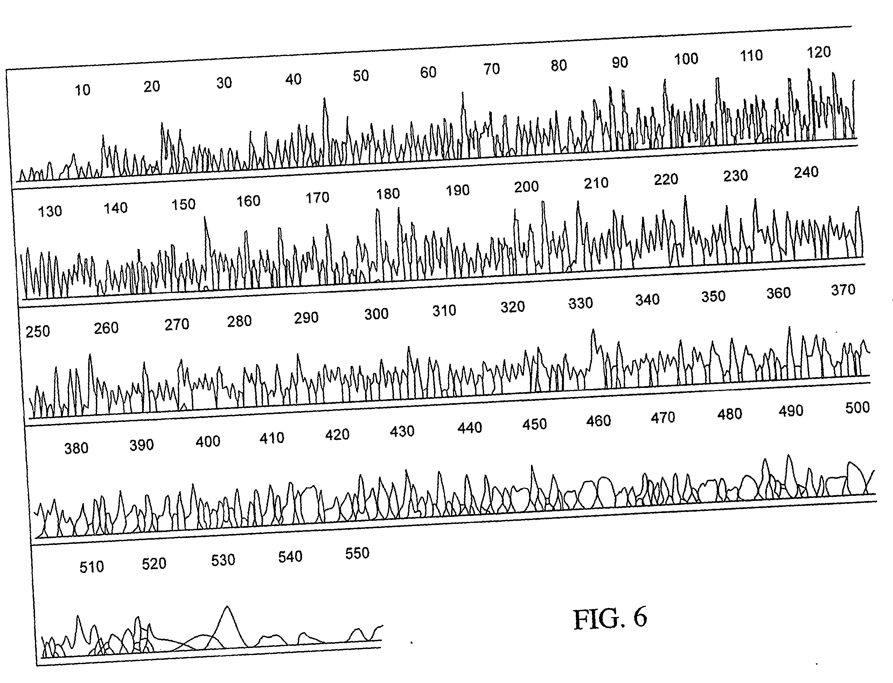

Fig. 6 illustrated the results from this purification. Fig. 6 illustrates that the synthetic polymer-polyelectrolyte-coated particles provided desirable purification. For example, poly(AA-co-DMA) polyelectrolyte-coated particles provided substantial removal of dye blob (residual dye-labeled ddNTPs) and relatively high signal strength indicating a desirable desalting of the sample.

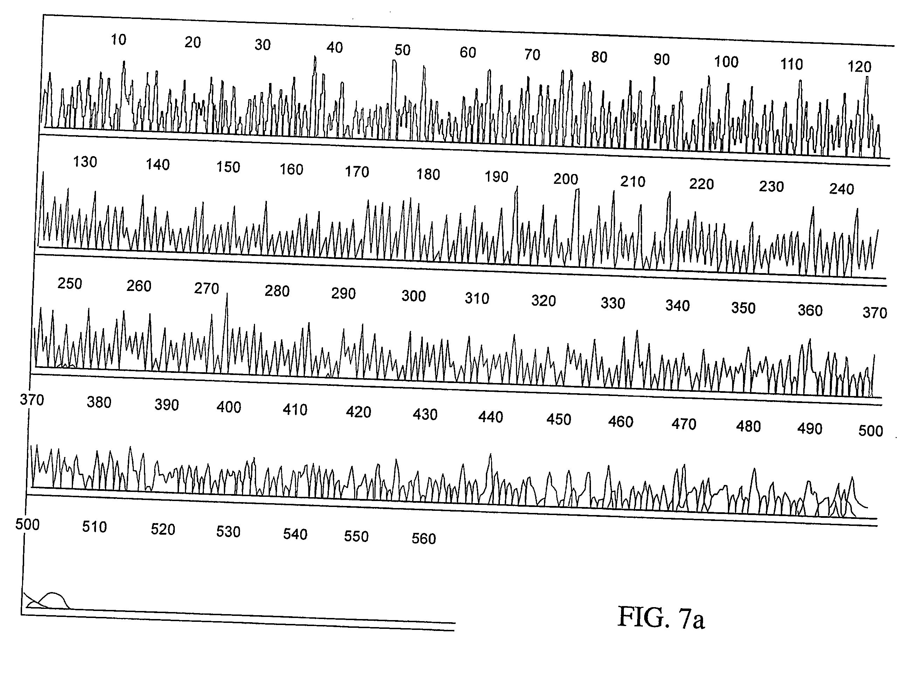

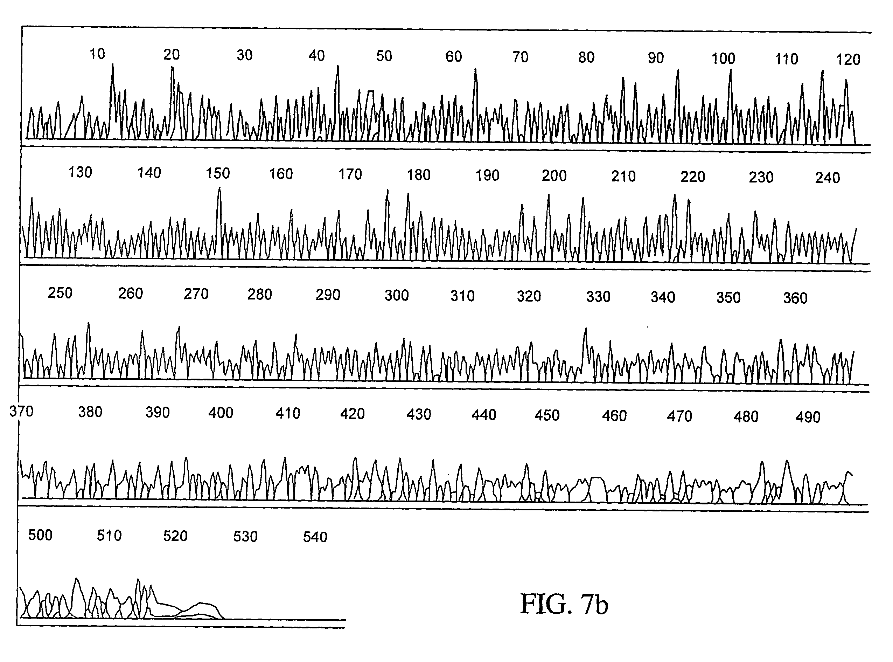

Other dRhodamine sequencing reaction products were purified by polyelectrolyte-coated particles. Fig. 7a illustrates the purification of sequencing reaction products by Bio-Rad AG 1-X8 ion-exchange resin coated with poly(AA-co-DMA) prepared as described herein Fig. 7b illustrates the purification of sequencing reaction products by Aminex A-27 ion-exchange resin coated with poly(AA-co-DMA) prepared as described herein The polyelectrolyte-coated particles provided substantial removal of dye blob (residual dye-labeled ddNTPs) and relatively high signal strength indicating a desirable desalting of the sample. Other ion-exchange resins, including Nucleosil, Isolute, Chromalite 30 SBG, Purolite-Chromalite, Macro-Prep Hi-Q, Bio-Rad AG 2-X8, Bio-Rad AG 1-X8, Aminex A-27, Powdex -PAO were tested with a variety of synthetic polymer polyelectrolytes for sequencing reaction product purification including poly(AA), poly(AA-co-AAm), poly(AA-co-DMA), poly(AA-co-PEOacrylate), poly(styrenesulfonic acid), poly(styrenesulfonic acid-co-DMA), and poly(AA-PEOacrylate-VSA).

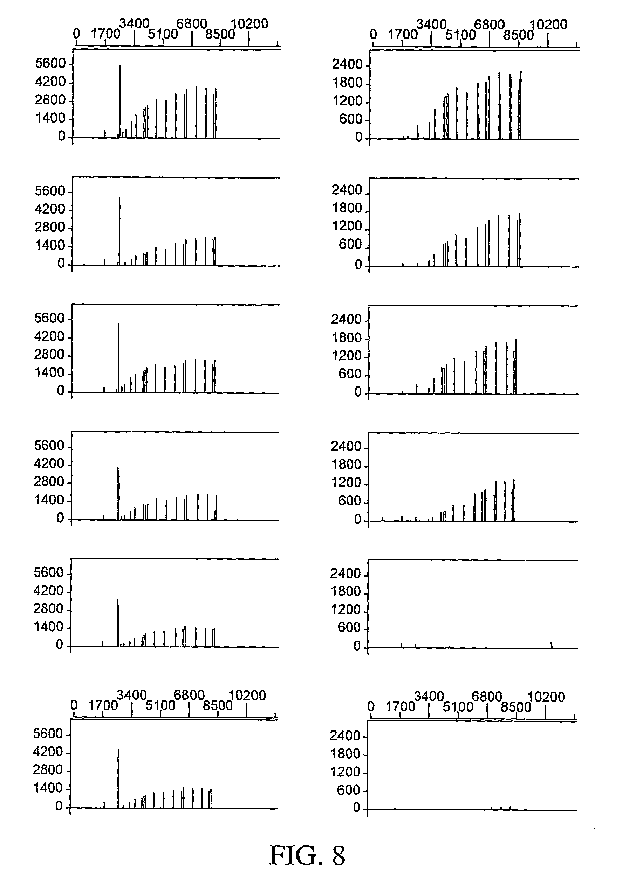

Fig. 8 illustrates the purification of sequencing reaction products purified by polyelectrolyte-coated particles coated with poly(AA-co-DMA). The sequencing reaction products were purified with different molecular weights of poly(AA-co-DMA) that increase from bottom to top electrophorograms for both the first column and second column. Sizing discrimination of polyelectrolyte-coated particles was evaluated using an assay based on the GeneScan® 500 ROX reagent (Applied Biosystems). The size standard consists of 16 dsDNA fragments ranging in size from 35bp to 500bp. The assay was performed by adding 5uL of the GeneScan® 500 ROX reagent to 5 ul of polyelectrolyte-coated particles. The mixture was agitated or vortexed for 5 minutes and the liquid is separated from the polyelectrolyte-coated particles. Separation of the polyelectrolyte-coated particles from the liquid was accomplished by centrifuging the mixture and pipetting the supernatant, or by filtration of the mixture. The resulting liquid was then analyzed using a DNA sequencer. Results of such an assay are shown in Fig. 8. The first column of Fig. 8 represents electrophorograms after separation by coated resins with pore sizes of 10 Angstroms to 15 Angstroms. The second column represents electrophorograms after separation by coated resins of pore size of 1000 Angstroms. The largest peak observed early in the electrophorogram is a primer peak and represents a fragment of 25 nucleotides. The electrophorograms in the first column of Fig. 8 shows no removal of small fragments for any of the molecular weights of coating, these electrophorograms are similar to the untreated control. Electrophorograms in the second column of Fig. 8 shows the elimination of the earlier peaks (smaller fragments) after separation with a lower molecular weight coating. These data demonstrate that the size cutoff for the polyelectrolyte-coated particles in the second column of Fig. 8 is 100bp, while the size cutoff of the polyelectrolyte-coated particles in the first column of Fig. 8 is less than 35bp. Fig. 8 illustrates the size cutoff for separation by the polyelectrolyte-coated particles for purification of sequencing reaction products.

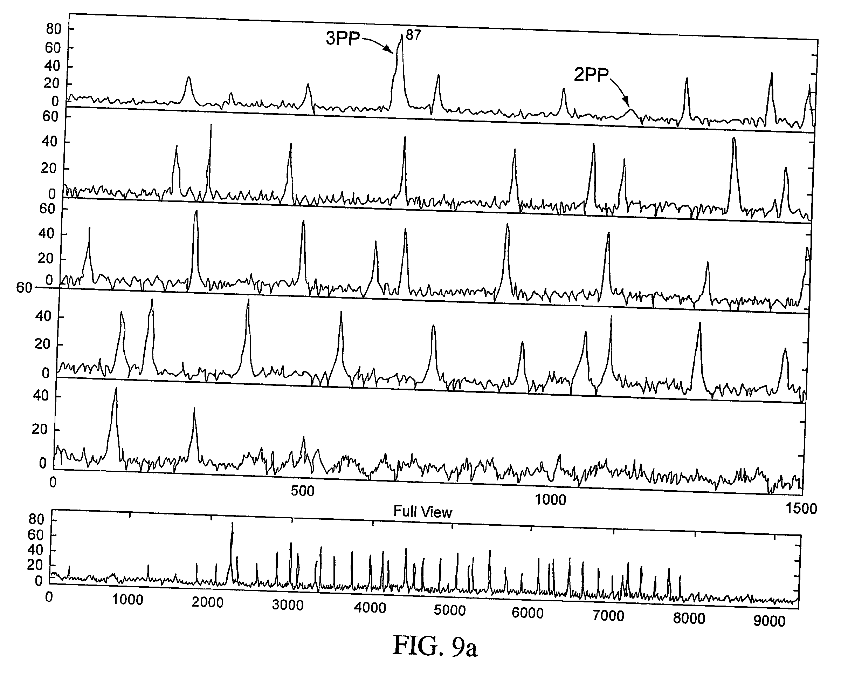

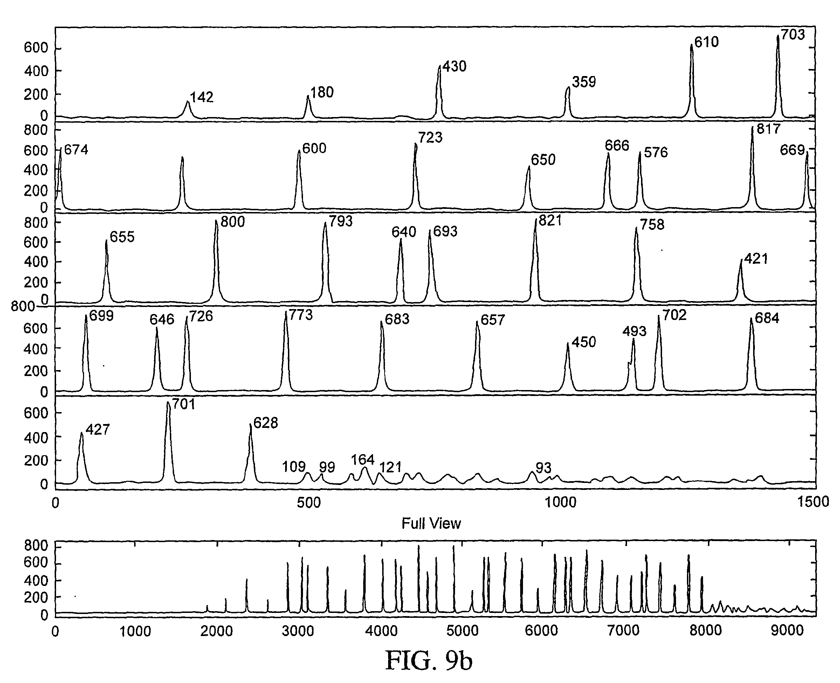

Figs. 9a and 9b illustrates the purification of sequencing reaction products purified by polyelectrolyte-coated particles including Powdex-PAO ion-exchange resin coated with poly(AA-co-DMA). Fig. 9a illustrates an electrophorogram taken before purification and Fig. 9b illustrates an electrophorogram taken after purification. The polyelectrolyte-coated particles removed LIZ® dye dTDP (2PP) and LIZ® dye dTTP (3PP) from the sequencing reaction products as labeled on Fig. 9a. The polyelectrolyte-coated particles removed other anions from the sequencing reaction products and improved electrokinetic injection of the sequencing reaction products, resulting in a factor of ten increase in signal strength.

### PCR REACTION PURIFICATION BY POLYELECTROLYTE-COATED PARTICLES WITH SYNTHETIC POLYMER:

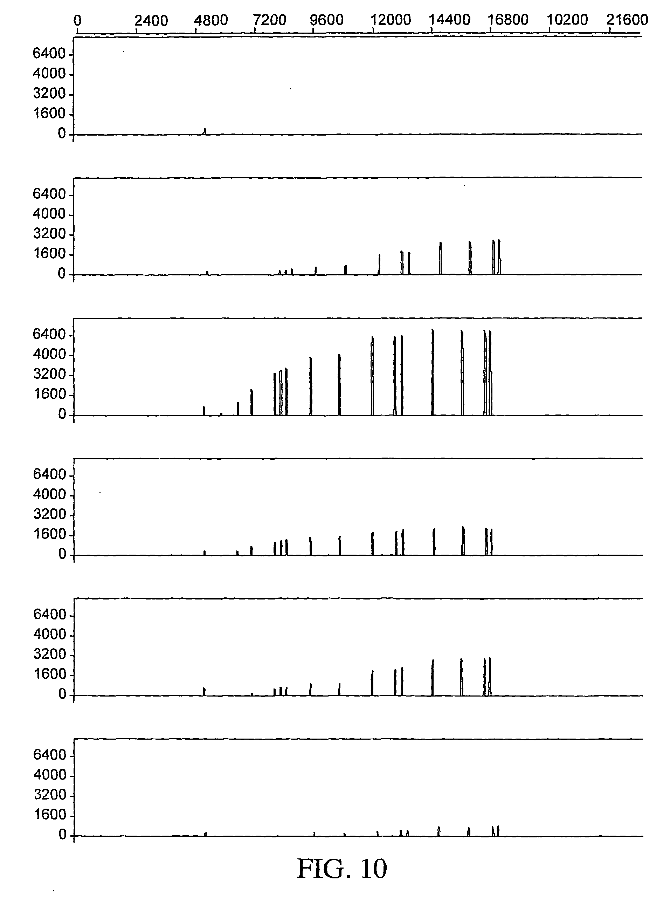

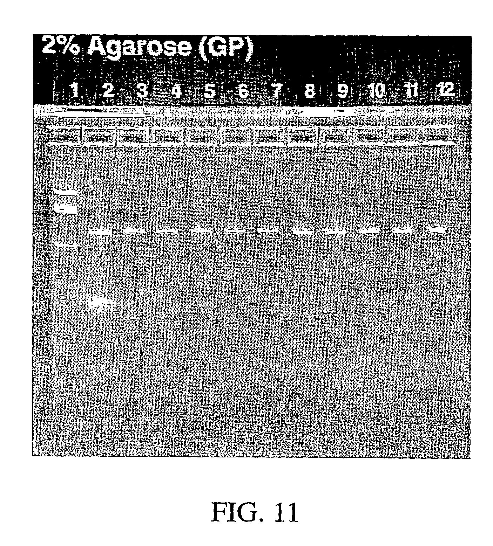

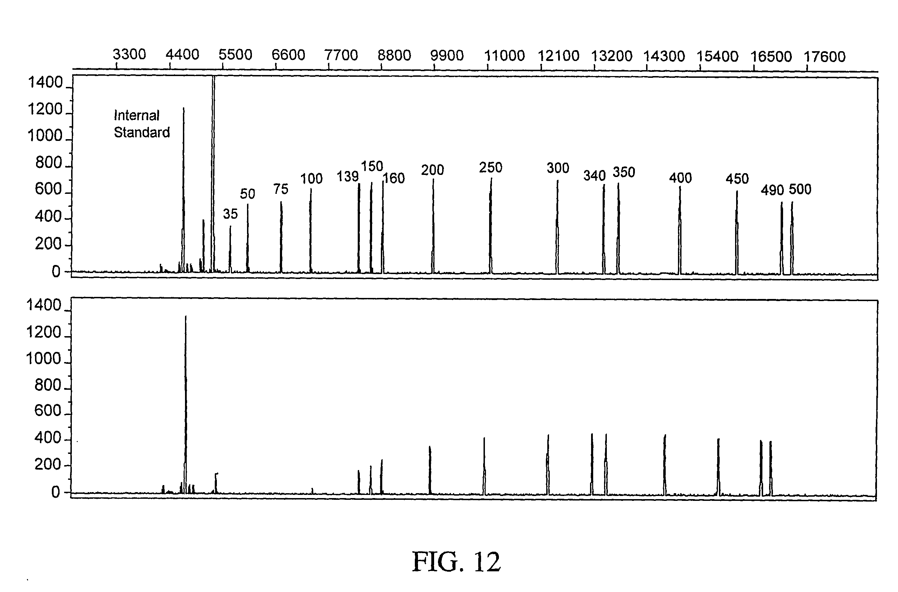

PCR reaction product purification was provided by polyelectrolyte-coated particles with synthetic polymer. Macro-Prep HQ ion-exchange resin was coated with poly(AA-co-DMA). Fig. 10 illustrates varying molar percentage of acrylic acid and molecular weigh of the poly(AA-co-DMA). The molecular weight and molar percentage increase from bottom to top from the bottom electrophorogram representing separation with resin coated with 1.1 mol% acrylic acid and 98.9 mol% DMA to the top electrophorogram representing separation with resin coated with 100 molar percent of acrylic acid or poly(AA) without N,N-dimethylacrylamide (DMA). Polyelectrolyte-coated particles containing 100 % acrylic acid removed primers, primer-dimer, and all DNA fragments. The same phenomenon was observed with poly(AA-co-DMA) containing a low acrylic acid content, 1.1 mol% acrylic acid. Fig. 11 illustrates the removal of oligonucleotide primers, primer-dimer and DNA fragments by non-desalting Macro-Prep 50 HQ (chloride form) ion-exchange resin coated with poly(AA-co-DMA) in the ranges described above. Lane 1 of Fig. 11 was loaded with the size standard, lane 2 was loaded with the one microliter of raw (no separation with polyelectrolyte coated ion-exchange particles) PCR product with 20 micromolar of primer, lanes 3-7 were loaded with one microliter of PCR product after separation with polyelectrolyte-coated ion-exchange particles, and lanes 8-12 were loaded with two microliters of PCR product after separation with polyelectrolyte-coated ion-exchange particles. Lane 2 shows the unseparated PCR products such as primers, primer-dimer, etc. as a diffuse band below the main band. Lanes 3-12 do not have such as corresponding band. Fig. 12 illustrates the size-based removal of primer-dimer and non-specifically amplified dsDNA from PCR products by non-desalting Macro-Prep 50 HQ (chloride form) ion-exchange resin coated with poly(AA-co-DMA) in the ranges described above. The upper electrophorogram shows PCR products with no separation with polyelectrolyte coated ion-exchange particles. The lower electrophorogram shows PCR products separated with polyelectrolyte coated ion-exchange particles. The difference illustrates that dsDNA fragments smaller than 100bp were separated from larger fragments that remain in solution after separation with polyelectrolyte coated ion-exchange particles.

### DNA SEQUENCING REACTION PURIFICATION BY POLYELECTROLYTE-COATED PARTICLES WITH SYNTHETIC POLYMER AND SURFACTANTS:

Surfactants can be added to DNA sequencing reactions prior to purification using size-based purification methods. For example, prior to purification of DNA sequencing reactions using size-exclusion spin columns, sodium dodecyl sulfate (SDS) can be added to assist in purification. Further, the DNA sequencing reaction including SDS can be heated prior to purification using size-exclusion spin columns. In various embodiments, anionic surfactants can be replaced with non-ionic surfactants in DNA sequencing reaction purified by polyelectrolyte-coated ion-exchange particles. Such replacement can prevent interaction of an ionic surfactant and the ion-exchange particle to avoid adverse effects on the ion-exchange capacity of the polyelectrolyte-coated ion-exchange particles. The term "non-ionic" surfactant as used herein refers to non-ionic surfactants, zwitter-ionic surfactants, or other surfactants that do not substantially contribute to the conductivity of the solution. Examples of non-ionic surfactants include Brij 35 and Brij 58 (CalBiochem, San Diego, CA), Triton X-100 (Sigma, St. Louis, MO), Octyl-β-D-glucoside, octylglucopyranoside, and (3-[(3-Cholamidopropyl) dimethylammonio]-1-propanesulfonate) ("CHAPS") (Pierce Biotechnology, Rockford, IL). In various embodiments, the non-ionic surfactants can be effective without heating in order to facilitate the removal of unicorporated dye-terminators from the DNA sequencing reaction.

For DNA sequencing reaction purification, a sample was prepared using fluorescently-labeled dideoxy nucleotide terminators so that the sequencing reaction product could be analyzed on a fluorescent capillary sequencer. A solution was prepared containing 20 uL using a 8 uL of Big Dye Terminator® v.3.1 Ready Reaction Mix (Applied Biosystems, Foster City, California). The sequencing primer was M13 universal reverse primer (3.2 pmol/uL). The template was a plasmid, pGEM (200 ng/uL). This master solution was aliquoted into wells in a GeneAmp® 9700 thermal cycler plate. The mixture was subjected to 25 cycles of heating, wherein each cycle included heating at 96°C for 10 seconds, heating at 50°C for 5 seconds, and heating at 60°C for 240 seconds.

After pooling, 10 uL of the pooled, unpurified reaction mixture was redistributed to each of 16 wells of a new 96 well MicroAmp® tray for purification. Each well contained the 10 uL of reaction mixture, 20 uL of de-ionized water, 10 uL of surfactant and 10 uL of polyelectrolyte-coated ion-exchange particle slurry including poly(styrenesulfonic acid)-coated AG1X-8 in a mixed bed with AG50WX-8 prepared as described above. The final volume of the mixture was 45 uL because of the polyelectrolyte-coated ion-exchange slurry was 50% water. In these examples, two different surfactant solutions were used: (1) 10 uL of Brij 35 aqueous solution at three percent by volume, final concentration of Brij 35 in entire solution was 0.67%; (2) and 10 uL of Triton X-100 aqueous solution at 10 percent by volume, final concentration of Triton X-100 in entire solution was 2.2%. Controls for each of the surfactants replaced the surfactant with 10 uL of water. In various embodiments, surfactants can be effective at concentrations ranging from 0.01% to 10%. In various embodiments, surfactants can be effective at concentrations ranging from 0.1% to 5%.

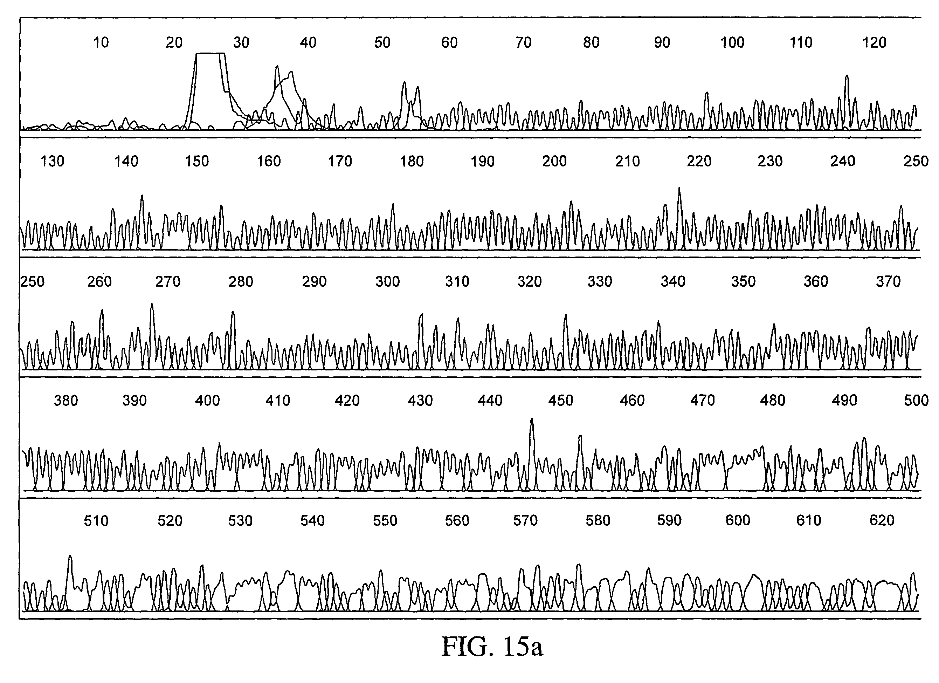

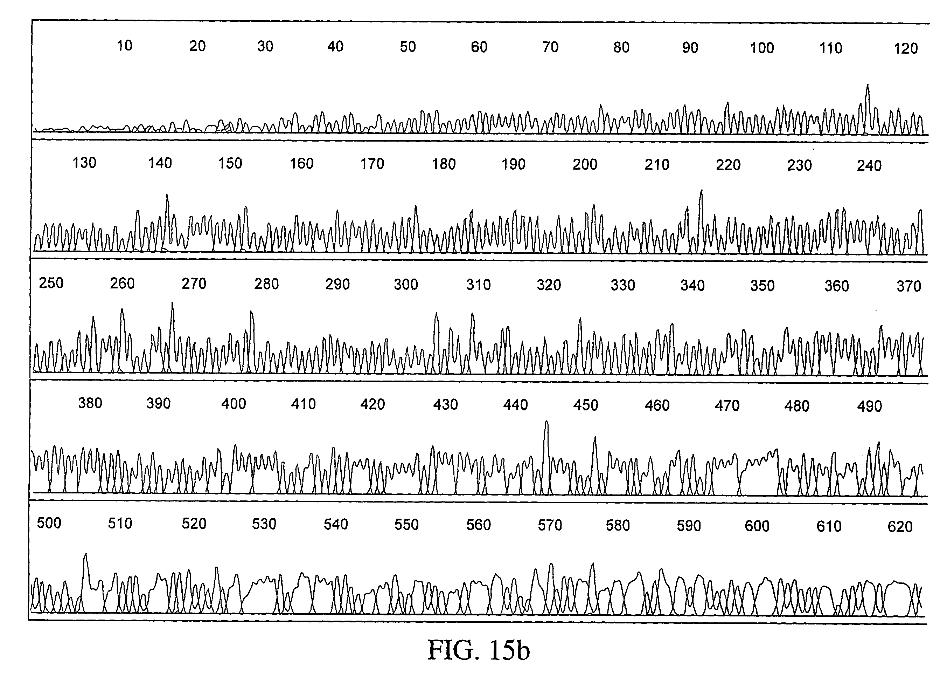

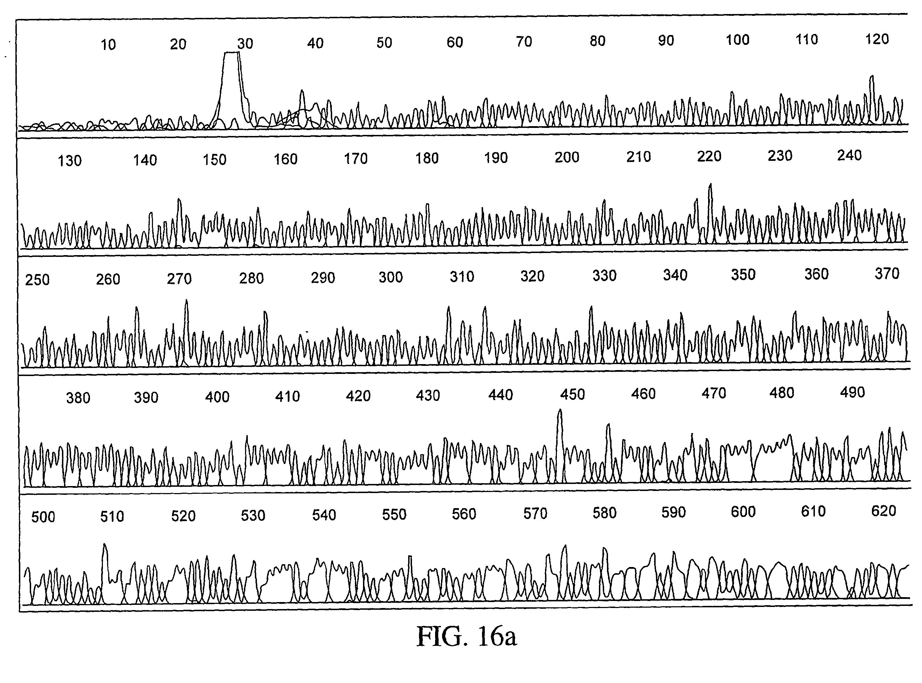

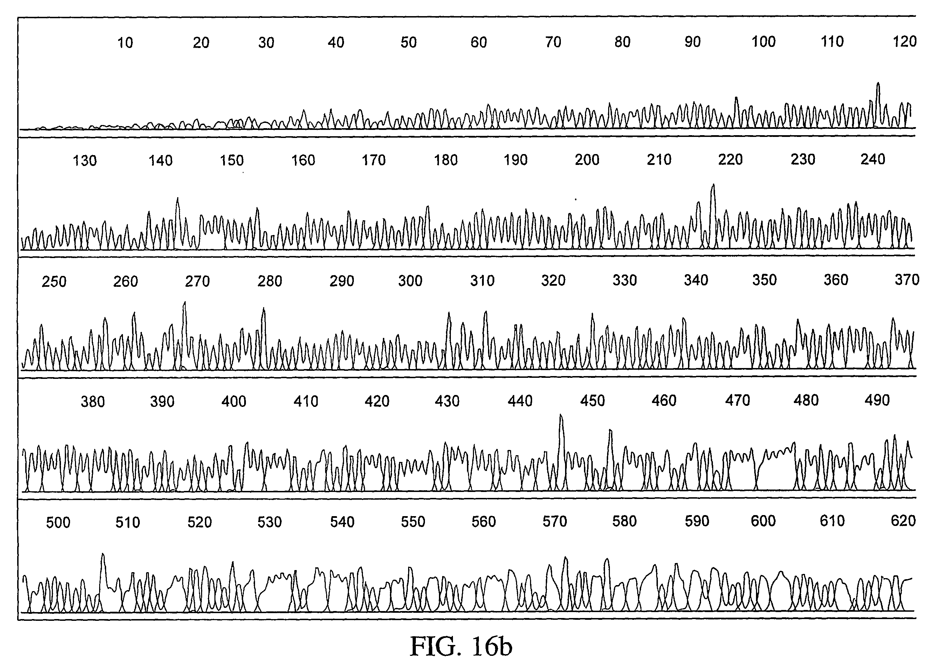

The samples in the MicroAmp® tray were then covered with an adhesive film and mixed with a vortexer for 30 minutes. Following mixing, the MicroAmp® tray containing the samples was centrifuged at 3000 rpm for five minutes. The MicroAmp® tray was then transferred to an ABI Prism® 3100 Genetic Analyzer for DNA sequencing. The DNA sequencing is carried out with a 50 cm capillary array and POP-6® capillary electrophoresis polymer. The injection conditions were 1000V at 22 seconds directly from the supernatant in the tube containing the sample and the beads as described below. Figs. 15A and 15B illustrate DNA sequencing purification with and without Brij 35 surfactant, respectively. In Fig. 15A, purified without Brij 35 surfactant, dye artifacts appeared, for example, in the region from 15-60 nucleotides of the sequence. In Fig. 15B, purified with Brij 35 surfactant, dye artifacts in that same region did not appear. Figs. 16A and 16B illustrate DNA sequencing purification with and without Triton X-100 surfactant, respectively. In Fig. 16A, purified without Triton X-100 surfactant, dye artifacts appeared, for example, in the region from 15-60 nucleotides of the sequence. In Fig. 16B, purified with Triton X-100 surfactant, dye artifacts in that same region did not appear. In both instances, the surfactant was able to facilitate removal of the dye artifacts without heating and without interfering with direct electrokinetic injection or polyelectrolyte-coated ion-exchange particle purification.

### COATING PARTICLES WITH MULTIPLE SYNTHETIC POLYMER LAYERS:

Poly(AA-co-DMA) polyelectrolyte was prepared as described above. Poly(allylamine hydrochloride), or "PAH," M_{w} 0.07 MDa and poly(acrylic acid), or "PAA," M_{w} 4.00 MDa (Aldrich Chemicals) were readily available. The following polyelectrolyte solutions (0.5 wt%) were prepared by adding the appropriate amount of polymers to the de-ionized waster and then tumbled overnight at ambient temperature: poly(AA-co-DMA) 0.2286g /39.915g H₂O; PAH 0.2268g 140.033g H₂O; PAA 0.1192 g / 19.99g H₂O.

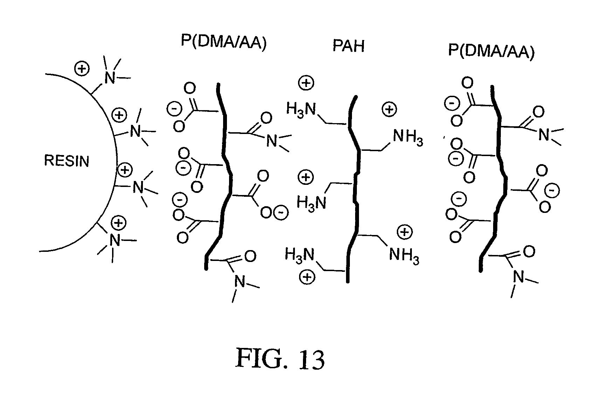

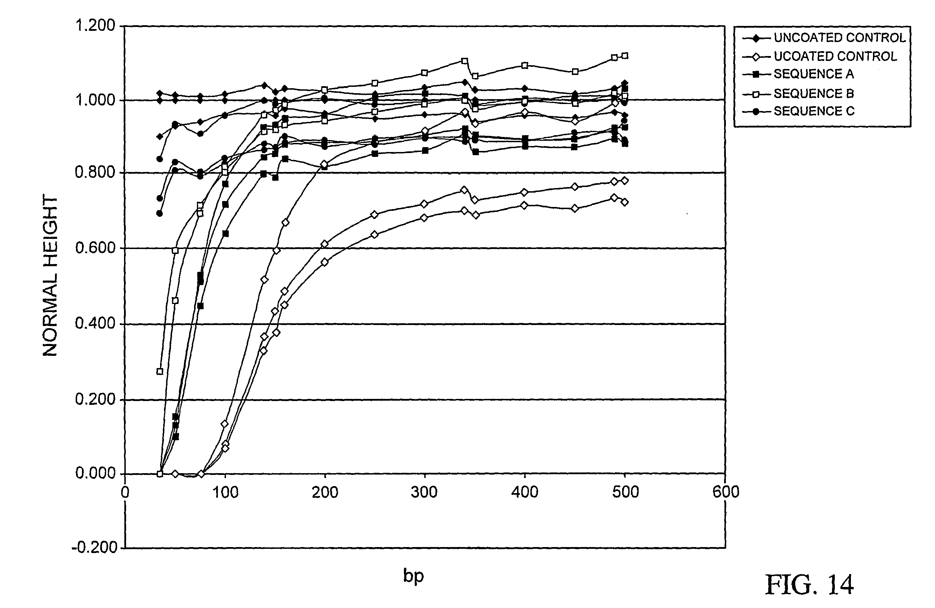

Prior to coating, the anion-exchange resin (Macro-Prep High Q 50, Bio-Rad) was washed with de-ionized water three times. A slurry solution of resin (3 mL, 0.35g of wet resin) and 20 mL of de-ionized water were added to a 50 mL tube. To coat the resin, the polyelectrolyte solution (0.750 mL) was added. The tube was vortexed for one minute, spun at 1760 rpm for three minutes, and the supernatant was decanted. The wet resin was dried under high vacuum oven at 70 degrees centigrade for two hours. This coating process was repeated two more times with subsequent polyelectrolyte solutions to apply a total of three polyelectrolyte coating layers. For the last drying step, the coating was dried overnight. The dried pellet was mixed by vortexing with 40 mL of de-ionized water, spun at 1670 rpm for three minutes, and the supernatant was removed. The final washed resin was redispersed in 10 mL of de-ionized water and stored in a refrigerator prior to use. Fig. 13 illustrates the multiple polyelectrolyte coatings applied to the resin in one of the examples. Fig. 14 illustrates the performance of an uncoated resin, a coated resin, and several trials of three sequences of multiple polyelectrolyte coatings on the ion-exchange resin by showing removal of smaller numbers of base pairs (bp). The three sequences of polyelectrolytes are described in Table 1 below, with the first layer being closest to the surface of the resin and subsequent layers after that.

**Table 1.**

| | Sequence A | Sequence B | Sequence C |
|---|---|---|---|
| First Layer | Poly(AA-co-DMA) | Poly(AA-co-DMA) | PAA |
| Second Layer | Poly(AA-co-DMA) | PAH | PAH |
| Third Layer | Poly(AA-co-DMA) | Poly(AA-co-DMA.) | PAA |

### MAGNETIZATION OF POLYELECTROLYTE COATED RESIN:

PAA M_{w} 4.00 MDa (Aldrich Chemicals) and Fe₃O₄ (EMG 1111) (Ferrotec Corp.) were readily available. A solution of PAA (0.750 mL) was added to a slurry solution of resin (0.2g of wet resin) and 10 mL de-ionized water. The mixture was vortexed for one minute, incubated at ambient temperature for five minutes, spun at 1760 rpm for three minutes, and the supernatant was decanted. The resin was washed once with de-ionized water (10 mL). The suspension was spun at 1760 rpm for three minutes and water was decanted. An aqueous solution of Fe₃O₄ (0.20 mL) with de-ionized water was added to the wet resin. The suspension was mixed by vortex for one minute and incubated at ambient temperature for five minutes. The suspension was spun at 1670 rpm for three minutes and the aqueous solution was removed. The resin was washed three times to remove excess unbound Fe₃O₄. The coated resin was redispersed in de-ionized water (10 mL). Magnetization of the resin was observed.

### SEPARATION OF POLYELECTROLYTE-COATED ION-EXCHANGE PARTICLES:

Within each category of reactions and/or examples provided above, there are numerous specific applications. For instance, PCR reaction polyelectrolyte-coated ion-exchange particles can provide purification for sequence detection applications (both endpoint and real-time), PCR cloning applications, etc., and DNA sequencing reaction polyelectrolyte-coated ion-exchange particles can provide purification for any type of nucleic acid sequencing, for example, sequencing, fragment analysis, single nucleotide polymorphism identification, etc.

It can be desirable to separate the polyelectrolyte-coated ion-exchange particles from the purified supernatant liquid before sequencing the nucleic acids. Sequencing can be achieved by, for example, capillary electrophoresis. Isolating the purified supernatant liquid from the polyelectrolyte-coated ion-exchange particles can add steps such as pipetting, sample transfers, centrifugation, magnetic removal and/or thermal cycling between purification and sequencing. It is desirable to eliminate such steps to increase efficiency and speed, and reduce cost and hands-on time for the user between purification and sequencing.

According to various embodiments, isolation of the polyelectrolyte-coated ion-exchange particles from the purified supernatant liquid can be achieved by filtration to retain the particles or centrifugation to collect the particles followed by aspiration of the supernatant liquid. It is desirable to eliminate the step of removing the polyelectrolyte-coated ion-exchange particles from the purified supernatant liquid by loading the sample for capillary electrophoresis directly from the supernatant liquid. However, the polyelectrolyte-coated ion-exchange particles can interfere with loading by inhibiting electrokinetic injection and causing random disruption in sequencing data or by clogging the capillary when polyelectrolyte-coated ion-exchange particles are crushed by the capillary against the bottom of the sample tube.

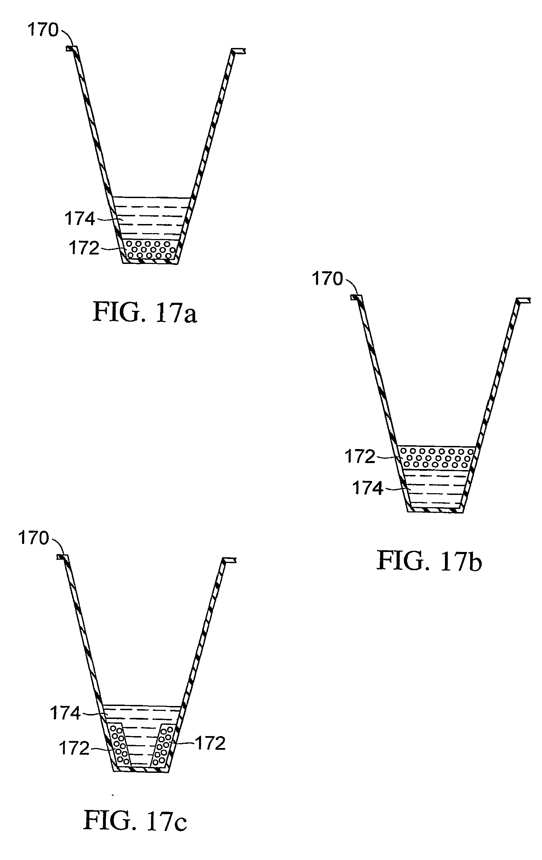

According to various embodiments, the present teachings provide a method for loading the capillary from the bottom of the sample well, tube, or chamber. The polyelectrolyte-coated ion-exchange particles can be moved to the sides or the top of the well. Figs. 17A-17C illustrate a well 170 where the polyelectrolyte-coated ion-exchange particles 172 have been isolated from the supernatant liquid 174. The teachings provide positioning the polyelectrolyte-coated ion-exchange particles 172 at the top of the well 170 as illustrated in Fig. 17B or positioning the polyelectrolyte-coated ion-exchange particles 172 at the sides of well 170 as illustrated in Fig. 17C.

According to various embodiments, the polyelectrolyte-coated ion-exchange particles can be positioned at the top of well by floating with a higher density solvent. The supernatant liquid's density can be increased such that it has greater density than the polyelectrolyte-coated ion-exchange particles. Examples of solvents that can increase the density of the supernatant include ethylene glycol (density = 1.113), propylene carbonate (density =1.189), polyethylene glycol (a waxy solid with density =1.127), glycerol (density = 1.261), and combinations thereof at various concentrations. In various embodiments, ion-exchange resins can have densities down to the 1.03 level. Upon increasing the density of the supernatant liquid, the polyelectrolyte-coated ion-exchange particles float to the top of the well. The capillary and electrode can then be immersed in the well below the layer of polyelectrolyte-coated ion-exchange particles. The loading of the capillary can be free of the polyelectrolyte-coated ion-exchange particles. The addition of the solvent eliminates centrifugation step in sample preparation and can be automated by an array pipettor.

According to various embodiments, the polyelectrolyte-coated ion-exchange particles can be positioned at the sides of the well by magnetic attraction. As described above, the polyelectrolyte-coated ion-exchange particles can be coated with Fe₃O₄ to provide magnetization. The polyelectrolyte-coated ion-exchange particles can be attracted to the sides of the well by a magnetic field on a side of the well or around the well provided by a magnet or an electrically induced magnetic field. The capillary and electrode can then be immersed in the center of the well clear of the concentric layer or beside the layer of polyelectrolyte-coated ion-exchange particles. The loading of the capillary can be free of the polyelectrolyte-coated ion-exchange particles. The magnetization of the polyelectrolyte-coated ion-exchange particles eliminates centrifugation step in sample preparation.

According to various embodiments, the present teachings provide a method for loading the capillary from the top of the sample well, tube, or chamber. The polyelectrolyte-coated ion-exchange particles can be moved to the bottom of the well. Fig. 17A illustrates a well 170 where the polyelectrolyte-coated ion-exchange particles 172 have been isolated from the supernatant liquid 174. The particles can be moved to the bottom by centrifugation or magnetization as discussed above. A sequencer instrument can be calibrated to load the sample from a higher point in the well free of the polyelectrolyte-coated ion-exchange particles. Modification of the capillary position for supernatant loading can be provided by increasing the distance between the capillary tip to the bottom of the tube. For standard microtiter trays, an example of such a distance is 4.5 mm. This corresponds to a well volume for a 96-well tray of 30 uL. The volume of the supernatant can be increased to insure that the capillary is immersed in supernatant for good electrokinetic injection. This can be done by increasing the volume of the supernatant by addition of de-ionized water. The mixture of the de-ionized water and the supernatant can be provided by vortexing the mixture for 30 minutes.

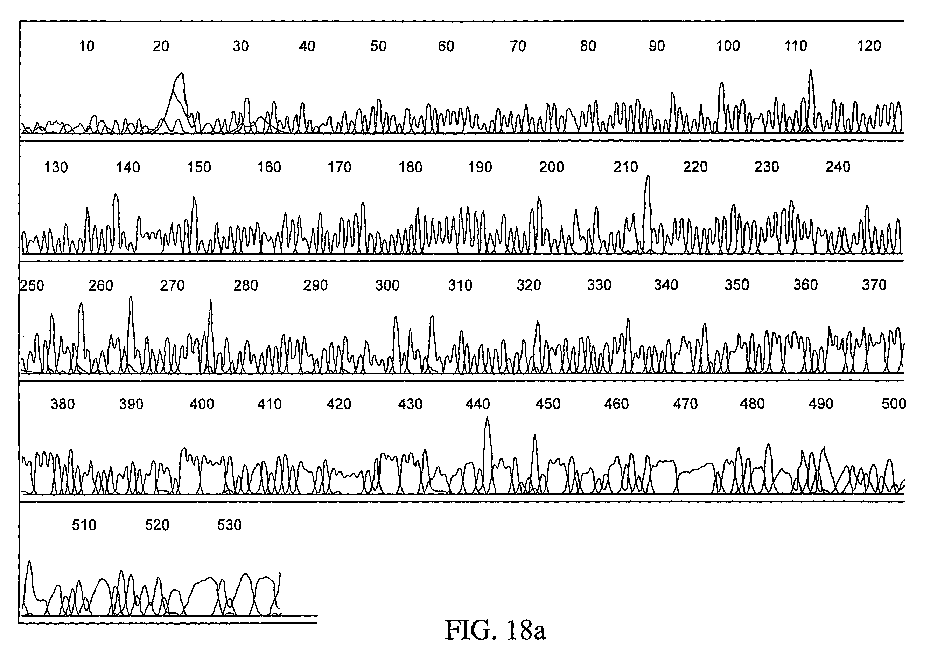

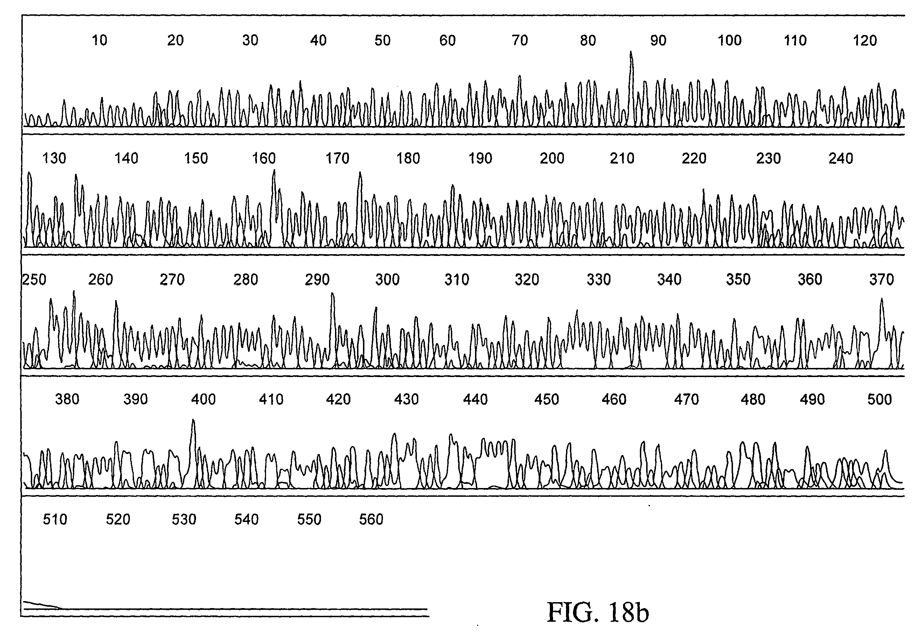

According to various embodiments, the sequencer instrument can be calibrated to load the sample from a higher point in the well by mechanically changing the tray or frame into which the tray mates so that the tray sits lower in the instrument. According to various embodiments, the sequencer instrument can be calibrated to load the sample from a higher point in the well by modifying the firmware initialization file to change the z-axis point-of-reference. According to various embodiments, the sequencer instrument can be calibrated to load the sample from a higher point in the well by programming software to retract the capillary from the bottom loading position to a point in the supernatant liquid layer above the polyelectrolyte-coated ion-exchange particles. Figs. 18A-18B illustrate nucleic acid sequencing from supernatant using Big Dye Terminator® chemistry (Fig. 18A) and dRhodamine terminator chemistry (Fig. 18B) using a sequencer instrument calibrated to load the sample from a higher point in the well by modifying the firmware initialization file to change the z-axis point-of-reference.

### COATING PARTICLES WITH SYNTHETIC POLYMERS:

According to various embodiments, the polyelectrolyte-coated ion-exchange particles can be coated with copolymers to select a desired cut-off for size-exclusion properties of the particles. The desired cut-off is a sharp decrease in amount of species below a certain size as shown by smaller numbers of base pairs (bp) in solution ("rolloff"). A rolloff assay to test cut-off measures the performance of the polyelectrolyte-coated ion-exchange particles with a fluorescently-labeled dsDNA size standard by illustrating the loss of fragments as a function of size using capillary electrophoresis. The peak height for each fragment can be normalized to a standard and a curve showing size discrimination. An example of a protocal can be using ROX-500 as a dsDNA size standard and LIZ-500 as an internal standard. The rolloff assay can indicate the loss of fragments during purification. Samples showing a low rolloff, do not show significant loss of low base pair fragments of the ROX-500 standard used in the assay. Samples showing a high rolloff, show high losses of the lower base pair fragments, for example 35, 50, 100, and 139 base pair fragments.

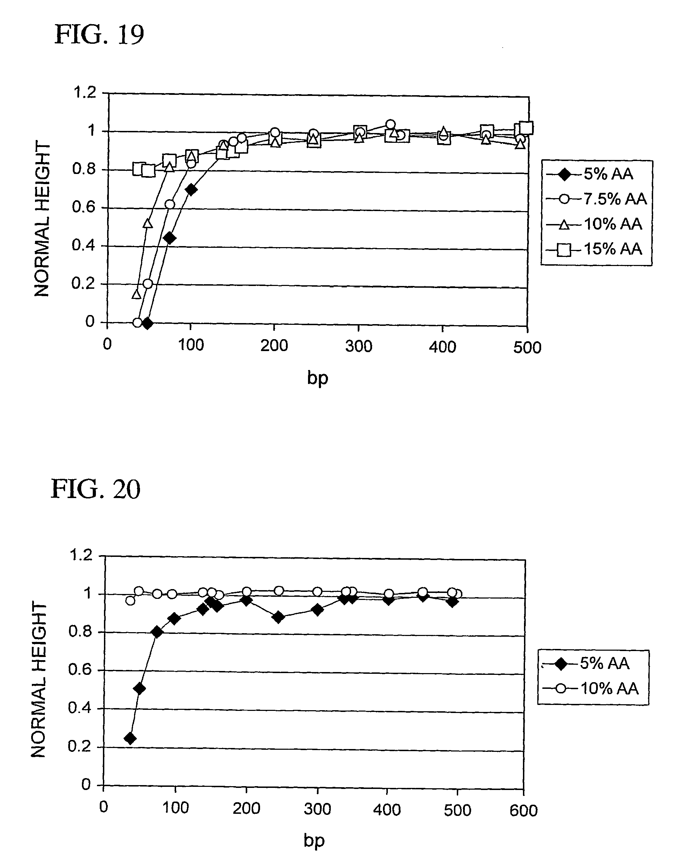

According to various embodiments, copolymers for coating the polyelectrolyte-coated ion-exchange particles can include those with those made from N,N-dimethylacrylamide (DMA) and acrylic acid (AA). As illustrated in Fig. 19, DMA-AA copolymers used to coat MP-1M BioRad ion-exchange resin demonstrated higher rolloff as the fraction of AA was decreased from 15%, 10%, 7.5%, and 5%.

According to various embodiments, copolymers for coating the polyelectrolyte-coated ion-exchange particles can include those with those made from acrylamide and AA. As illustrated in Fig. 20, acrylamide-AA copolymers use to coat MP-1M BioRad ion-exchange resin demonstrated higher rolloff as the fraction of AA was decreased from 10% and 5%.

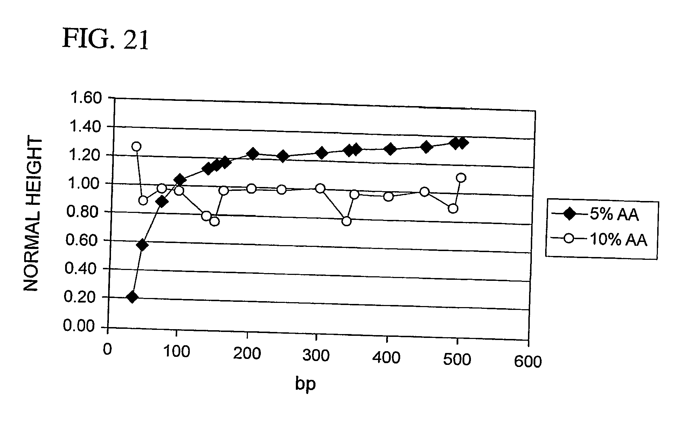

According to various embodiments, copolymers for coating the polyelectrolyte-coated ion-exchange particles can include those with those made from 2-hydroxyethylacrylate (HEA) and AA. As illustrated in Fig. 21, HEA-AA copolymers use to coat MP-1M BioRad ion-exchange resin demonstrated higher rolloff as the fraction of AA was decreased from 10% and 5%.

According to various embodiments, copolymers can enhance rolloff performance and shelf life of the treated ion-exchange resin. Co-agents can be water-soluble or slightly water-soluble homopolymers or copolymers prepared by monomers including, for example (meth)acrylamide, N-methyl(methyl)acrylamide, N,N-dimethyl(methyl)acrylamide, N-ethyl(meth)acrylamide, N-n-propyl(meth)acrylamide, N-iso-propyl(meth)acrylamide, N-ethyl-N-methyl(meth)acrylamide, N,N-diethyl(meth)acrylamide, N-hydroxymethyl(meth)acrylamide, N-(3-hydroxypropyl)(meth)acrylamide, N-vinylformamide, N-vinylacetanide, N-methyl-N-vinylacetamide, vinyl acetate that can be hydrolyzed to give vinylalcohol after polymerization, 2-hydroxyethyl(meth)acrylate, 3-hydroxypropyl(meth)acrylate, N-vinylpyrrolidone, poly(ethylene oxide)(meth)acrylate, N-(meth)acryloxysuccinimide, N-(meth)acryloylinorpholine, N-2,2,2-trifluoroethyl(meth)acrylamide, N-acetyl(meth)acrylamide, N-amido(meth)acrylamide, N-acetamido(meth)acrylamide, N-tris(hydroxymethyl)methyl(meth)acrylamide, N-(methyl)acryloyltris(hydroxymethyl)methylamine, (methyl)acryloylurea, vinyloxazolidone, vinylmethyloxazolidone, or a combination thereof, and copolymerized with acrylic acid.

### DNA SEQUENCING REACTION PURIFICATION BY POLYELECTROLYTE-COATED PARTICLES WITH SYNTHETIC POLYMER, SURFACTANTS, AND STABILIZING ADDITIVES:

DNA sequencing reaction products can be purified prior to separation by capillary electrophoresis (CE) in a DNA sequencer. The purification can remove unicorporated dye-labeled dideoxynycleotides that can interfere with separation by CE. Injection into a CE system should be from a solution of substantially low ionic strength, for example 1mM salt, in order to provide adequate injections. DNA sequencers with CE can be automated instruments that can store and process multiple trays of DNA sequencing samples. These systems can form a queue of samples sets in a storage area of an automated DNA sequencer for up to 24 hours before processing. Most existing purification methods cannot maintain the purification media in contact with sample sets longer than eight hours to avoid breakdown. It is desirable to provide a DNA sequencing reaction purification with polyelectrolyte-coated particles that can be stable against degradation for longer than eight hours, such as 48 hours.

It is desirable to provide DNA sequencing reaction products in deionized aqueous injection solution that are stable and resist degradation without inhibiting electrokinetic injection of DNA sequencing products into a CE system. Other methods of stabilizing such as addition of ethylenediaminetetracetic acid (EDTA) increase the ionic strength of the DNA sequencing product solution and thereby inhibiting electrokintetic injection. It is desirable to purify the DNA sequencing reactions with a mixed bed of polyelectrolyte-coated ion-exchange particles. The mixed bed can maintain the ionic strength of the DNA sequencing solution substantially low ionic strength. Stabilizers that are ionic can be absorbed by the particles and removed from the solution. It is desirable to stabilize the DNA sequencing solution with non-ionic materials that are not removed from solution by the polyelectrolyte-coated ion-exchange particles.

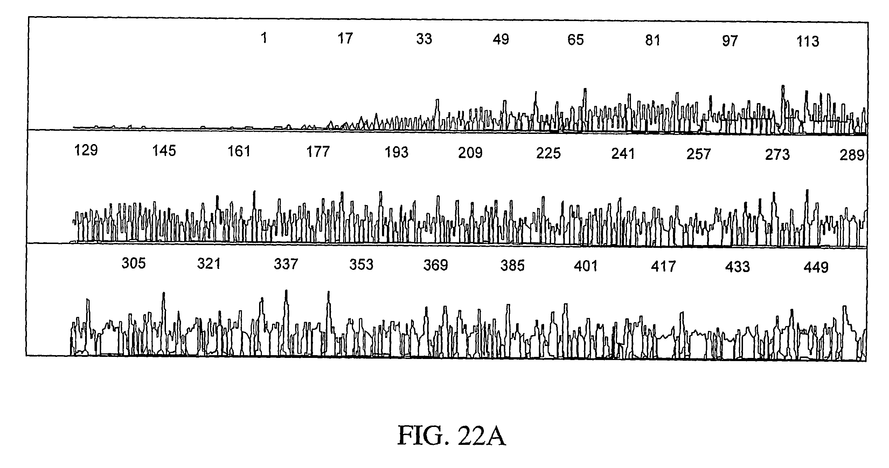

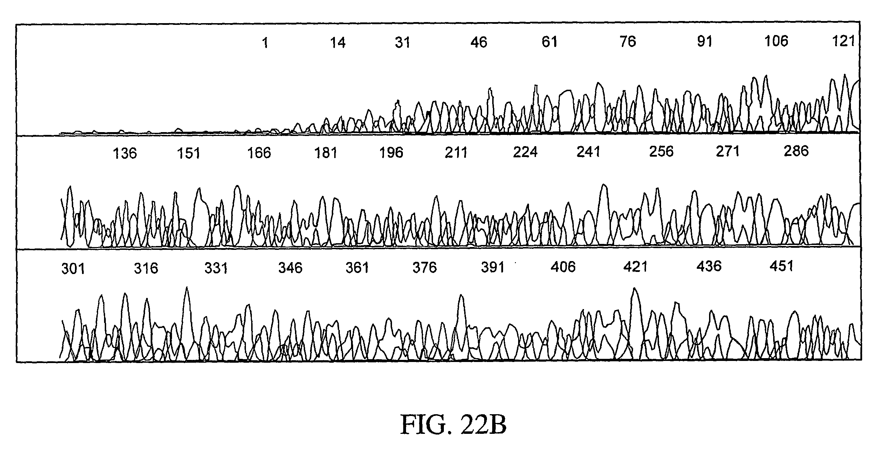

Electrokinetic injection solutions for CE systems can include DI water or deionized formamide (DI formamide). EDTA has been used as a stabilizer for DNA sequencing product solutions with DI formamide. DTT with or without the addition of sodium bicarbonate has been used as a stabilizer for DNA sequencing product solutions with formamide to resist fluorescent dye breakdown. Instability of DNA sequencing reaction products in DI water has been considered insubstantial. However, DNA sequencing reaction products stored in DI water can become destabilized and breakdown. For example, DNA sequencing reaction products stored 24 hours in DI water show substantial peak broadening when analyzed. As illustrated in Fig. 22A, a DNA sequencing reaction in DI water at three hours showed insubstantial peak broadening. Whereas, as illustrated in Fig. 22B, DNA sequencing reaction products in DI water at 24 hours showed substantial peak broadening. It is desirable to use DI water in the DNA sequencing product solution injected into the CE because DI water is inexpensive and non-toxic, DI water provides higher signal strength than injections from DI formamide, and DI water is compatible with polyelectrolyte-coated ion-exchange particles.

According to various embodiments, zwitter-ionic compounds can resist degradation of DNA sequencing reaction products in DI water. For example, 1-cabroxymethyl-N,N,N,-trimethyl-methanaminium hydroxide inner salt (betaine) can stabilize DNA sequencing reactions in DI water forming a stable aqueous solution for electrokinetic injection. Zwitter-ionic compounds can provide substantially neutral, substantially low conductivity, and/or insubstantially ionic conditions for electrokinetic injection into CE systems. Zwitter-ionic compounds can be treated as neutral by the polyelectrolyte-coated ion-exchange particles used in purification of DNA sequencing reaction products and are not substantially absorbed by the particles as are other ionic species such as a salts.

According to various embodiments, the stabilizing additive can be introduced to DNA sequencing reaction products with purification media, for example polyelectrolyte-coated ion-exchange particles, etc., or to DNA sequencing reaction products that have already been purified by other means, for example ethanol precipitation, isopropanol precipitation, spin-columns, polyelectrolyte-coated ion-exchange particles, etc. and then separated from the purification media.

According to various embodiments, as described above, addition of surfactants to DNA sequencing reaction products prior to purification can enhance the removal of dye artifacts when the sample is subsequently separated by CE in a DNA sequencer. In various embodiments, the surfactant can be in the DNA sequencing reaction product solution with a stabilizing additive and polyelectrolyte-coated ion-exchange particles. The surfactant can be anionic, cationic, non-ionic, zwitter-ionic or a combination of these types of surfactants. The surfactant can be added at a concentration from 0.01% to 10%, or 0.5% to 5%, or 1% to 10%. The stabilizing additive can be a zwitterions, for example betaine, a sulfur-containing compound, for example dithiothreitol, an oxidizable phenol, for example catechol, a monosaccharide, disaccharide, polysaccharide, or amino acid. The stabilizing additive can be at a concentration 1mM to 5M, or 0.5M to 2M, or 0.1 mM to 250 mM, or 1M to 3M.

According to various embodiments, the surfactant can include octyl phenol ethoxylate (Triton X-100), polyoxyethylleneglycol dodecyl ether (Brij 35), polyethylene glycol hexadecyl ether (Brij 58), octylphenoxylpolyethoxyethanol (NP-40), Brij 97, polyoxythylnesorbitan monolaurate (Tween 20, Tween 80), 3-(decyldimethylammonio)propanesulfonate inner salt (Zwittergent 3-8, 3-12, 3-14), 3-[3-(cholamidoprooyl)-dimethylammonio]propanesulfonate (CHAPS), 3-[3-(cholamidoprooyl)-dimethylammonio]-2-hydroxy-1-propanesulfonate (CHAPSO), N,N-bis[3-D-glucoamido)propyl]cholamide (Big CHAP), detergent sulfobetaine (C7BzO), Tergitol (NP 7, 9, 11, 13, 15), N-(alkylC10-C16)-N,N-dimethylglycine betaine (Empigen BB), n-dodecyl maltoside, Luberol PX, n-octyl glucoside, 3-(4-Heptyl)phenyl-3-hydroxypropyl-dimethylammonio- propane sulfonate (C7BzO), nondetergent sulfobetaines, or combinations thereof.

According to various embodiments, the stabilizing additive can include 1-cabroxymethyl-N,N;N,-trimethyl-methanaminium hydroxide (betaine), methylglycine, dimethylglycine, 4-(2-hydroxyethyl)piperazine-1-propanesulfonic acid (EPPS), N-[tris(hyrdoxymethyl)methyl]glycine (tricine), N,N-bis(2-hydroxyethyl)glycine (bicine), N-[tris(hydroxymethyl)methyl]-3-aminopropanesulfonic acid (TAPS), Good's buffers, 2-mecaptoethanol, DTT, tris(2-carboxylethyl)phosphine hydrochloride (TCEP), trehalose, mannitol, sorbitol, and combinations thereof.

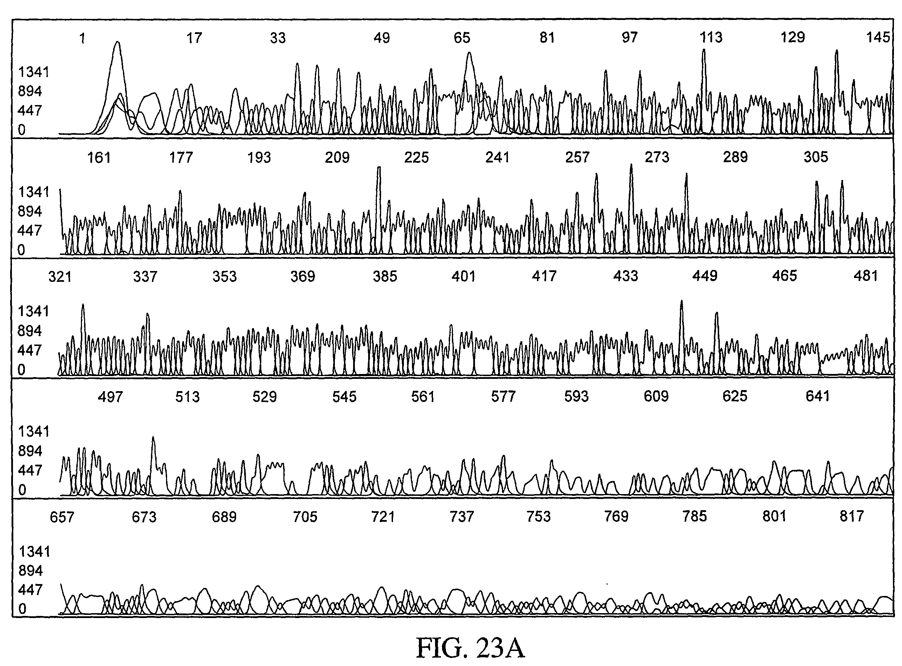

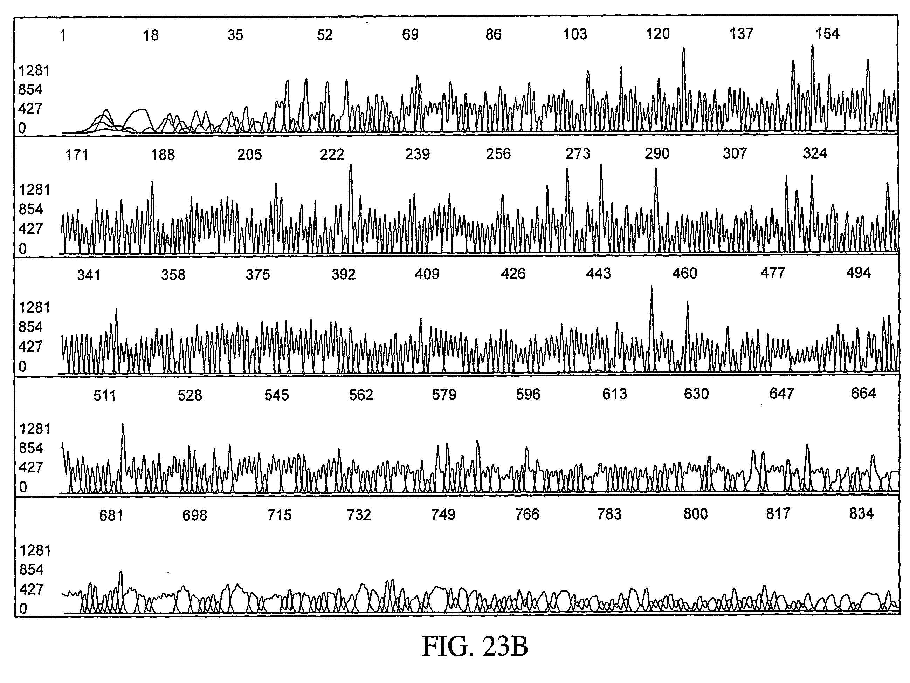

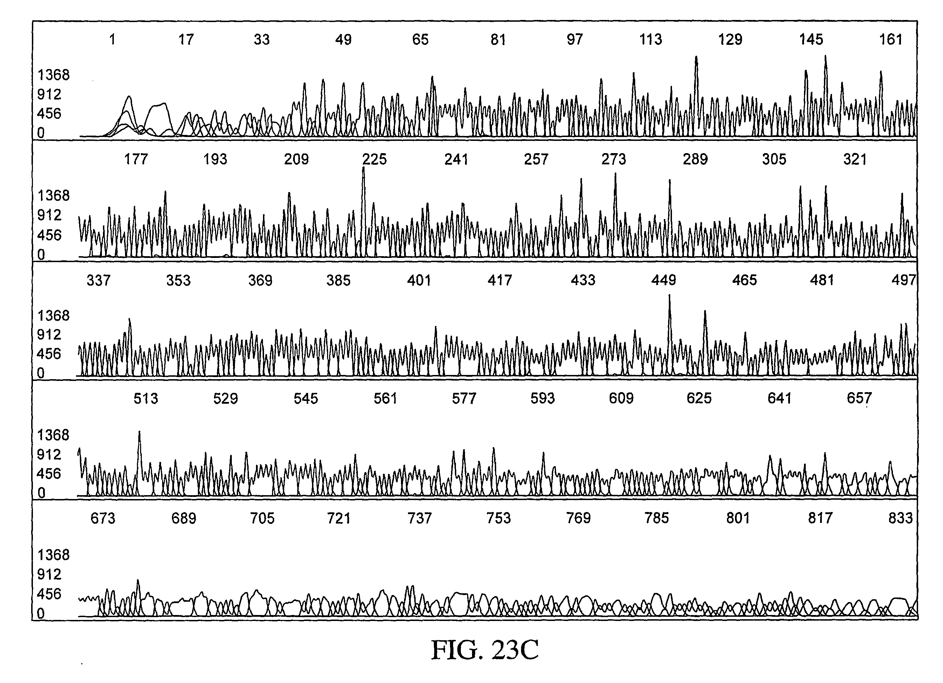

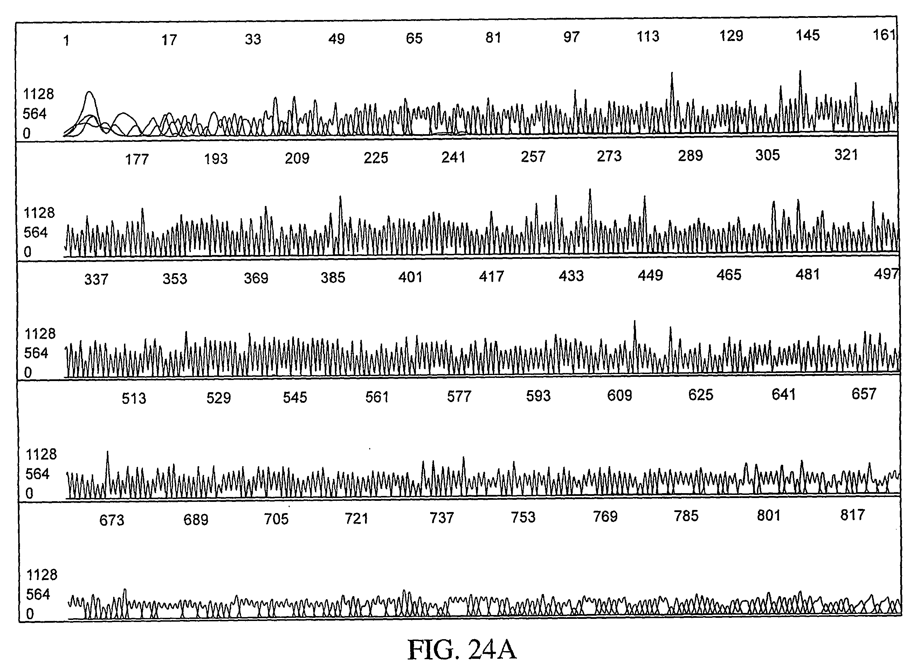

Figs. 23A-23C and 24A-24C show the DNA sequencer electropherogram results generated by an ABI PRISM 3100 Genetic Analyzer (Applied Biosystems, Foster City, CA) with experiments illustrating the present inventions. Fig. 23A shows an electropherogram where the DNA sequencing reaction products with 1M betaine and 2.5mM DTT that were purified without a surfactant. An undesirable dye artifact was observed interfering with the basecalling at near base 70. Fig. 23B shows the same reaction products that were purified using 3% Triton X-100 in addition to the stabilization additives, betaine and DTT. The addition of Triton X-100 resulted in an electropherogram with no dye artifacts. Fig. 23C shows the same reaction products that were purified using 3% Brij 35 in addition to the stabilizing additives, betaine and DTT. The addition of Brij 35 resulted in an electropherogram with no dye artifacts. Alternative experiments have shown that DTT similar results without DTT.

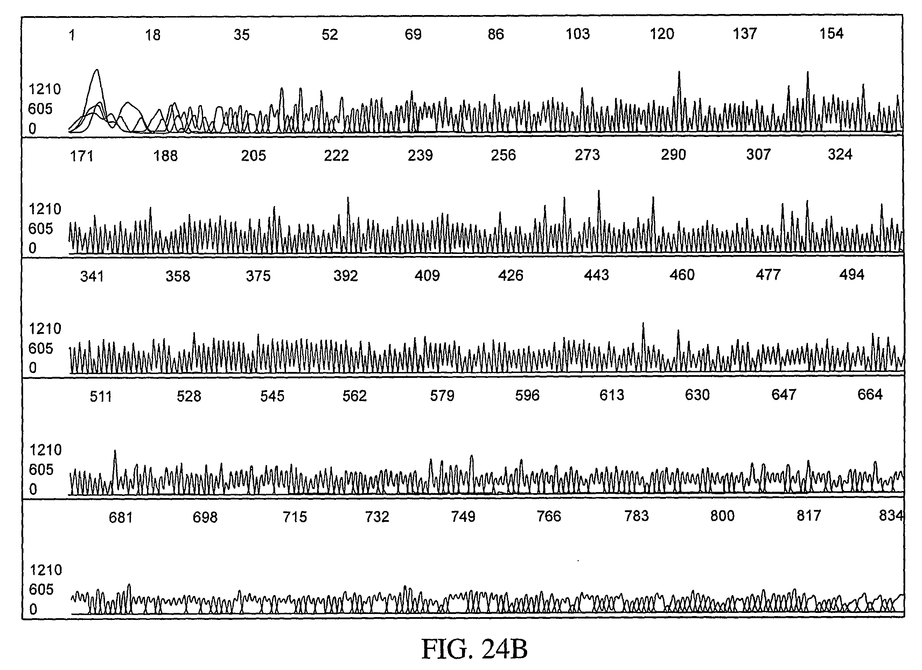

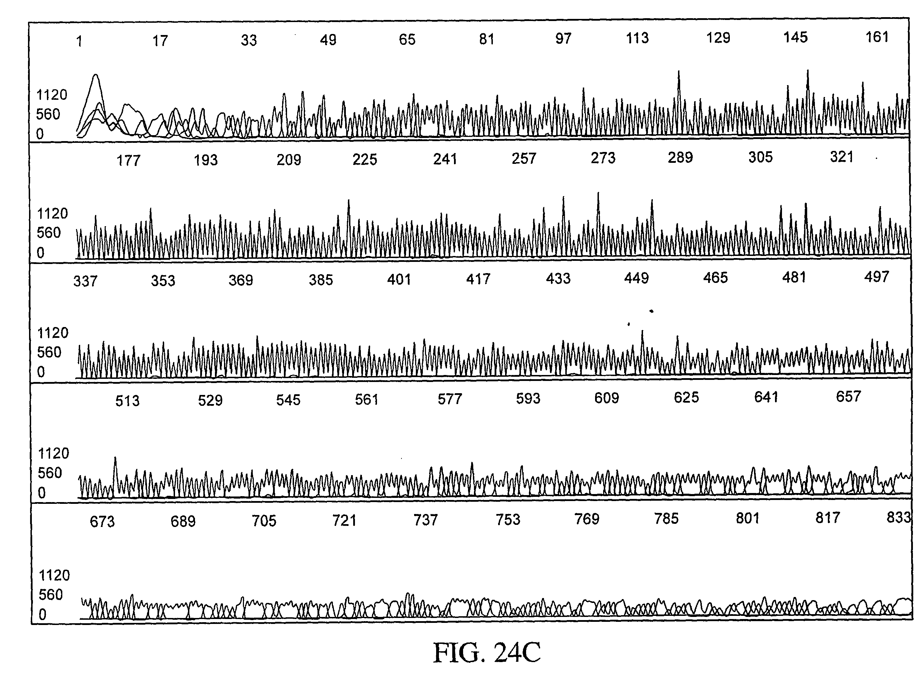

Figs. 24A-24C show electropherograms when the DNA sequencing reaction products were combined with 1.5M betaine, 2.5mM DTT and 3% CHAPS and while this solution was maintained in contact with the polyelectrolyte-coated ion-exchange particles that were used as purification media. The solutions were incubated for various times. Fig. 22B illustrated that in the absence of additives, the quality of the DNA sequencing electropherogram deteriorates. Fig. 24A shows the electropherogram that resulted from the solution that was incubated at room temperature (20 degrees Celsius) for six hours. Compared to the degradation observed in Fig. 22B, the peak resolution in Fig. 24A was good with the sequence readable up to 800 nuclotides and no dye artifacts were observed in the early part of the sequence. Figs. 24B and 24C show the same reaction incubated for 24 and 48 hours, respectively. Even at 24 and 48 hours there was not significant degradation of the quality of the sequencing reaction detection. These examples demonstrated the ability of the stabilizing additives to enhance removal of dye artifacts and stabilize the DNA sequencing reactions in a deionized aqueous solution, even in the presence of polyelectrolyte-coated ion-exchange particles.

According to various embodiments, a method for purification with polyelectrolyte-coated ion-exchange particles can include addition of CHAPS and betaine to sequencing reaction products, addition of mixture of polyelectrolyte-coated ion-exchange particles and uncoated ion-exchange particles, sealing of solution, mixing for 30 to 60 minutes, centrifugation, and supernatant injection.

For the purposes of this specification and appended claims, unless otherwise indicated, all numbers expressing quantities of ingredients, percentages or proportions of materials, reaction conditions, and other numerical values used in the specification and claims, are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

It is noted that, as used in this specification and the appended claims, the singular forms "a," "an," and "the," include plural referents unless expressly and unequivocally limited to one referent. Thus, for example, reference to "a monomer" includes two or more monomers. Furthermore, the use of the term "including", as well as other forms, such as "includes" and "included", is not limiting.

## Claims

1. A solution comprising: a particle, the particle comprising: a core comprising ion-exchange material; and a coating covering the outer surface of the ion-exchange core wherein the coating comprises polyelectrolyte material, wherein the core and coating are adapted to separate DNA sequencing reaction products and wherein the polyelectrolyte creates a size exclusion barrier allowing small molecules which have a size of less than 300 bp to penetrate into the particle and restricting larger molecules from interacting with the core; a non-ionic surfactant; and a stabilizing additive which stabilizes DNA sequencing reactions in a deionized aqueous solution and which is a non-ionic material that is not removed from solution by the polyelectrolyte-coated ion-exchange particle.

2. The solution of claim 1, wherein the small molecules have a size of less than 100 bp.

3. The solution of claim 1 or 2, wherein the small molecules are primers, primer-dimers and DNA fragments.

4. The solution of claim 1, wherein the small molecules include inorganic buffer ions and nucleotides.

5. The solution of claim 1, wherein the larger molecules are dye-labeled single stranded DNA fragments having greater than 45 nucleotides.

6. The solution of claim 1. wherein the larger molecules are single stranded DNA having a size of 10 nucleotides or greater.

7. The solution of claim 1, wherein the non-ionic surfactant is selected from the group consisting of octyl phenol ethoxylate (Triton X-100), polyoxyethyleneglycol dodecyl ether (Brij 35), polyethylene glycol hexadecyl ether (Brij 58), octylphenoxylpolyethoxyethanol (NP-40), Brij 97, polyoxyethylenesorbitan monolaurate (Tween 20, Tween 80), 3-(decyldimethylammonio)-propanesulfonate inner salt (Zwittergent 3-8, 3-12, 3-14), 3-[3-(cholamidopropyl)-dimethylammonio]propanesulfonate (CHAPS), 3-[3-(cholamidopropyl)-dimethylammonio]-2-hydroxy-1-propanesulfonate (CHAPSO), N,N-bis[3-D-glucoamido)propyl]cholamide (Big CHAP), detergent sulfobetaine (C7BzO), Tergitol (NP 7, 9, 11, 13, 15), N-(alkylC10-C16)-N,N-dimethylglycine betaine (Empigen BB), n-dodecyl maltoside, Luberol PX, n-octyl glucoside, 3-(4-Heptyl)phenyl-3-hydroxypropyl-dimethylammonio-propane sulfonate (C7BzO), nondetergent sulfobetaines, and combinations thereof.

8. The solution of claim 7, wherein the non-ionic surfactant is CHAPS.

9. The solution of claim 1, wherein the stabilizing additive is a zwitter-ionic compound, a sulfur-containing compound, an oxidizable phenol, a monosaccharide, disaccharide, a polysaccharide, or an amino acid.

10. The solution of claim 9, wherein when the stabilizing additive is a zwitter-ionic compound then the zwitter-ionic compound is betaine.

11. The solution of claim 1, wherein the non-ionic surfactant is effective without heating in order to facilitate the removal of unincorporated dye-terminators from the DNA sequencing reaction.

12. The solution of claim 1, wherein the ion-exchange material is cationic.

13. The solution of claim 12, further comprising an anion-exchange particle.

14. The solution of claim 1, wherein the ion-exchange material is anionic.

15. The solution of claim 14, further comprising a cation-exchange particle.

16. The solution of claim 1, wherein the polyelectrolyte material is not crosslinked.

17. A method for DNA sequencing, the method comprising: providing a purification media, contacting the purification media comprising the solution of claim 1 with DNA sequencing reaction products to remove unincorporated dye-terminators and salts; isolating the purification media; and loading a sample for capillary electrophoresis from the DNA sequencing products without interference from the purification media.

18. The method of claim 17, wherein the loading comprises electrokinetic injection.

19. The method of claim 17, wherein the purification media comprises a plurality of particles, and wherein isolating comprises magnetically manipulating the plurality of particles, floating the plurality of particles, or applying a centrifugal or gravitational force to the plurality of particles.

20. The method of claim 17, further comprising providing a non-ionic surfactant, wherein the non-ionic surfactant is selected from the group consisting of octyl phenol ethoxylate (Triton X-100), polyoxyethyleneglycol dodecyl ether (Brij 35), polyethylene glycol hexadecyl ether (Brij 58), octylphenoxylpolyethoxyethanol (NP-40), Brij 97, polyoxyethylenesorbitan monolaurate (Tween 20, Tween 80), 3-(decyldimethylammonio)-propanesulfonate inner salt (Zwittergent 3-8, 3-12, 3-14), 3-[3-(cholamidopropyl)-dimethylammonio]propanesulfonate (CHAPS), 3-[3-(cholamidopropyl)-dimethylammonio]-2-hydroxy-1-propanesulfonate (CHAPSO), N,N-bis[3-D-glucoamido)propyl]cholamide (Big CHAP), detergent sulfobetaine (C7BzO), Tergitol (NP 7, 9, 11, 13, 15), N-(alkylC10-C16)-N,N-dimethylglycine betaine (Empigen BB), n-dodecyl maltoside, Luberol PX, n-octyl glucoside, 3-(4-Heptyl)phenyl-3-hydroxypropyl-dimethylammonio-propane sulfonate (C7BzO), nondetergent sulfobetaines, and combinations thereof.

21. The method of claim 17, wherein the stabilizing additive is betaine.

22. The method of claim 17, further comprising storing the DNA sequencing reaction products, the purification media, and the stabilizing additive for at least eight hours without destabilizing the DNA sequencing reaction products.

## Patentansprüche

1. Lösung, die umfasst: ein Partikel, wobei das Partikel umfasst: einen Kern, der ein Ionenaustauschmaterial umfasst; sowie eine Beschichtung, die die äußere Oberfläche des Ionenaustauschkerns bedeckt, wobei die Beschichtung ein Polyelektrolytmaterial umfasst, wobei der Kern und die Beschichtung angepasst sind, um DNA-Sequenzierungs-Reaktionsprodukte zu trennen, und wobei der Polyelektrolyt eine Größenausschlussgrenze schafft, die kleinen Molekülen, die eine Größe von weniger als 300 bp aufweisen, erlaubt, in das Partikel einzudringen und größere Moleküle von der Interaktion mit dem Kern ausschließt; ein nicht-ionisches Tensid; sowie einen stabilisierenden Zusatzstoff, der DNA-Sequenzierungsreaktionen in einer deionisierten wässrigen Lösung stabilisiert und der ein nicht-ionisches Material ist, das durch das Polyelektrolytbeschichtete Austauschmaterial nicht aus der Lösung entfernt wird.

2. Lösung gemäß Anspruch 1, wobei die kleinen Moleküle eine Größe von weniger als 100 bp aufweisen.

3. Lösung gemäß Anspruch 1 oder 2, wobei die kleinen Moleküle Primer, Primer-Dimere und DNA-Fragmente sind.

4. Lösung gemäß Anspruch 1, wobei die kleinen Moleküle anorganische Pufferionen und Nukleotide aufweisen.

5. Lösung gemäß Anspruch 1, wobei die größeren Moleküle Farbstoff-beladene Einzelstrang-DNA-Fragmente mit mehr als 45 Nukleotiden sind.

6. Lösung gemäß Anspruch 1, wobei die größeren Moleküle einzelsträngige DNA mit einer Größe von 10 Nukleotiden oder mehr sind.

7. Lösung gemäß Anspruch 1, wobei das nicht-ionische Tensid ausgewählt wird aus der Gruppe bestehend aus Octylphenolethoxylat (Triton X-100), Polyoxyethylenglycoldodecylether (Brij 35), Polyethylenglycolhexadecylether (Brij 58), Octylphenoxylpolyethoxyethanol (NP-40), Brij 97, Polyoxyethylensorbitanmonolaurat (Tween 20, Tween 80), inneres Salz von 3-(Decyldimethylammonium)-propansulfonat (Zwittergent 3-8, 3-12, 3-14), 3-[3-(Cholamidpropyl)-dimethylammonium]-propansulfonat (CHAPS), 3-[3-(Cholamidpropyl)-dimethylammonium]-2-hydroxy-1-propansulfonat (CHAPSO), N,N-Bis[3-D-gluconamid)-propyl]-cholamid (Big CHAP), Detergens-Sulfobetain (C7BzO), Tergitol (NP 7, 9, 11, 13, 15), N-(AlkylC₁₀-C₁₆)-N,N-dimethylglycinbetain (Empigen BB), n-Dodecylmaltosid, Luberol PX, n-Octylglucosid, 3-(4-Heptyl)-phenyl-3-hydroxypropyldimethylammoniumpropan-sulfonat (C7BzO), Nicht-Detergens-Sulfobetainen und Kombinationen davon.

8. Lösung gemäß Anspruch 7, wobei das nicht-ionische Tensid CHAPS ist.

9. Lösung gemäß Anspruch 1, wobei der stabilisierende Zusatzstoff eine zwitterionische Verbindung, eine schwefelhaltige Verbindung, ein oxidierbares Phenol, ein Monosaccharid, Disaccharid, ein Polysaccharid oder eine Aminosäure ist.

10. Lösung gemäß Anspruch 9, wobei, wenn der stabilisierende Zusatzstoff eine zwitterionische Verbindung ist, die zwitterionische Verbindung Betain ist.

11. Lösung gemäß Anspruch 1, wobei das nicht-ionische Tensid ohne Erwärmen wirksam ist, um das Entfernen von nichteingebauten Dye-Terminatoren aus der DNA-Sequenzierungsreaktion zu erreichen.

12. Lösung gemäß Anspruch 1, wobei das Ionenaustauschmaterial kationisch ist.

13. Lösung gemäß Anspruch 12, weiterhin umfassend ein Anionenaustauschpartikel.

14. Lösung gemäß Anspruch 1, wobei das Ionenaustauschmaterial anionisch ist.

15. Lösung gemäß Anspruch 14, weiterhin umfassend ein Kationenaustauschpartikel.

16. Lösung gemäß Anspruch 1, wobei das Polyelektrolytmaterial nicht quervernetzt ist.

17. Verfahren zur DNA-Sequenzierung, wobei das Verfahren umfasst: Bereitstellen eines Reinigungsmediums, in Kontakt bringen des Reinigungsmediums, das die Lösung gemäß Anspruch 1 mit DNA-Sequenzierungsreaktionsprodukten umfasst, um nicht eingebaute Dye-Terminatoren und Salze zu entfernen; Abtrennen des Reinigungsmediums; sowie Laden einer Probe für die Kapillar-Elektrophorese von den DNA-Sequenzierungsprodukten ohne Interferenz von Seiten des Reinigungsmediums.

18. Verfahren gemäß Anspruch 17, wobei das Laden elektrokinetische Injektion umfasst.

19. Verfahren gemäß Anspruch 17, wobei das Reinigungsmedium eine Vielzahl von Partikeln umfasst, und wobei das Abtrennen das magnetische Manipulieren der Vielzahl von Partikeln, das Aufschlämmen der Vielzahl von Partikeln oder das Anwenden einer Zentrifugal- oder Schwerkraft an der Vielzahl der Partikel umfasst.

20. Verfahren gemäß Anspruch 17, weiterhin umfassend das Bereitstellen eines nicht-ionischen Tensids, wobei das nicht-ionische Tensid ausgewählt wird aus der Gruppe bestehend aus Octylphenolethoxylat (Triton X-100), Polyoxyethylenglycoldodecylether (Brij 35), Polyethylenglycolhexadecylether (Brij 58), Octylphenoxylpolyethoxyethanol (NP-40), Brij 97, Polyoxyethylensorbitanmonolaurat (Tween 20, Tween 80), inneres Salz von 3-(Decyldimethylammonium)-propansulfonat (Zwittergent 3-8, 3-12, 3-14), 3-[3-(Cholamidpropyl)-dimethylammonium]-propansulfonat (CHAPS), 3-[3-(Cholamidpropyl)-dimethylammonium]-2-hydroxy-1-propansulfonat (CHAPSO), N,N-Bis[3-D-gluconamid)-propyl]-cholamid (Big CHAP), Detergens-Sulfobetain (C7BzO), Tergitol (NP 7, 9, 11, 13, 15), N-(AlkylC₁₀-C₁₆)-N,N-dimethylglycinbetain (Empigen BB), n-Dodecylmaltosid, Luberol PX, n-Octylglucosid, 3-(4-Heptyl)-phenyl-3-hydroxypropyldimethylammoniumpropan-sulfonat (C7BzO), Nicht-Detergens-Sulfobetainen und Kombinationen davon.

21. Verfahren gemäß Anspruch 17, wobei der stabilisierende Zusatzstoff Betain ist.

22. Verfahren gemäß Anspruch 17, weiterhin umfassend das Aufbewahren der DNA-Sequenzierungsreaktionsprodukte, der Reinigungsmedien und des stabilisierenden Zusatzstoffes für mindestens acht Stunden ohne Destabilisieren der DNA-Sequenzierungsreaktionsprodukte.

## Revendications

1. Solution comprenant : une particule, la particule comprenant : un noyau comprenant un matériau échangeur d'ions ; et un enrobage couvrant la surface externe du noyau échangeur d'ions où l'enrobage comprend un matériau polyélectrolytique, où le noyau et l'enrobage sont adaptés pour séparer les produits de la réaction de séquençage de l'ADN et où les polyélectrolytes créent une barrière d'exclusion par la taille permettant aux petites molécules d'une taille inférieure à 300 bp de pénétrer dans la particule et empêchant les molécules plus grosses d'interagir avec le noyau ; un tensioactif non ionique ; et un additif stabilisant qui stabilise les réactions de séquençage de l'ADN dans une solution aqueuse désionisée et qui est un matériau non ionique qui n'est pas éliminé de la solution par la particule échangeuse d'ions enrobée de polyélectrolytes.

2. Solution selon la revendication 1, dans laquelle les petites molécules ont une taille inférieure à 100 bp.

3. Solution selon la revendication 1 ou 2, dans laquelle les petites molécules sont des amorces, des dimères d'amorces et des fragments d'ADN.

4. Solution selon la revendication 1, dans laquelle les petites molécules comprennent des ions de tampon minéraux et des nucléotides.

5. Solution selon la revendication 1, dans laquelle les molécules plus grosses sont des fragments d'ADN simple brin marqués par un colorant comportant plus de 45 nucléotides.

6. Solution selon la revendication 1, dans laquelle les molécules plus grosses sont de l'ADN simple brin ayant une taille de 10 nucléotides ou supérieure.

7. Solution selon la revendication 1, dans laquelle le tensioactif non ionique est choisi dans le groupe constitué d'éthoxylate d'octyl-phénol (Triton X-100), d'éther de polyoxyéthylène glycol et de dodécyle (Brij 35), d'éther de polyéthylène glycol et d'hexadécyle (Brij 58), d'octylphénoxylpolyéthoxyéthanol (NP-40), de Brij 97, de monolaurate de polyoxyéthylène sorbitane (Tween 20, Tween 80), de sel interne de 3-(décyldiméthylammonio)-propanesulfonate (Zwittergent 3-8, 3-12, 3-14), de 3-[3-(cholamidopropyl)-diméthylammonio]propanesulfonate (CHAPS), de 3-[3-(cholamidopropyl)-diméthylammonio]-2-hydroxy-1-propanesulfonate (CHAPSO), de N,N-bis[3-D-glucoamido)propyllcholamide (Big CHAP), de sulfobétaïne détergente (C7BzO), de Tergitol (NP 7, 9, 11, 13, 15), de N-(alkyl en C₁₀ à C₁₆)-N,N-diméthylglycine bétaïne (Empigen BB), de n-dodécyl-maltoside, de Luberol PX, de n-octyl-glucoside, de 3-(4-heptyl)phényl-3-hydroxy-propyl-diméthylammonio-propane sulfonate (C7BzO), de sulfobétaïnes non détergentes et de combinaisons de ceux-ci.

8. Solution selon la revendication 7, dans laquelle le tensioactif non ionique est le CHAPS.

9. Solution selon la revendication 1, dans laquelle l'additif stabilisant est un composé zwittérionique, un composé contenant du soufre, un phénol oxydable, un monosaccharide, un disaccharide, un polysaccharide ou un acide aminé.

10. Solution selon la revendication 9, dans laquelle lorsque l'additif stabilisant est un composé zwittérionique alors le composé zwittérionique est une bétaïne.

11. Solution selon la revendication 1, dans laquelle le tensioactif non ionique est efficace sans chauffage afin de faciliter l'élimination des nucléotides de terminaison marqués par un colorant non incorporés provenant de la réaction de séquençage de l'ADN.

12. Solution selon la revendication 1, dans laquelle le matériau échangeur d'ions est cationique.

13. Solution selon la revendication 12, comprenant en outre une particule échangeuse d'anions.

14. Solution selon la revendication 1, dans laquelle le matériau échangeur d'ions est anionique.

15. Solution selon la revendication 14, comprenant en outre une particule échangeuse de cations.

16. Solution selon la revendication 1, dans laquelle le matériau polyélectrolytique n'est pas réticulé.

17. Procédé de séquençage d'ADN, le procédé comprenant : la fourniture d'un milieu de purification, la mise en contact du milieu de purification comprenant la solution selon la revendication 1 avec les produits de la réaction de séquençage de l'ADN pour éliminer les nucléotides de terminaison marqués par un colorant non incorporés et les sels ; l'isolement du milieu de purification ; et le chargement d'un échantillon pour une électrophorèse capillaire à partir des produits de séquençage de l'ADN sans interférence du milieu de purification.

18. Procédé selon la revendication 17, dans lequel le chargement comprend une injection électrocinétique.

19. Procédé selon la revendication 17, dans lequel le milieu de purification comprend une pluralité de particules, et dans lequel l'isolement comprend la manipulation de manière magnétique de la pluralité de particules, la mise en flottaison de la pluralité des particules ou l'application d'une force centrifuge ou gravitationnelle à la pluralité de particules.

20. Procédé selon la revendication 17, comprenant en outre la fourniture d'un tensioactif non ionique, dans lequel le tensioactif non ionique est choisi dans le groupe constitué d'éthoxylate d'octyl-phénol (Triton X-100), d'éther de polyoxyéthylène glycol et de dodécyle (Brij 35), d'éther de polyéthylène glycol et d'hexadécyle (Brij 58), d'octylphénoxylpolyéthoxyéthanol (NP-40), de Brij 97, de monolaurate de polyoxyéthylène sorbitane (Tween 20, Tween 80), de sel interne de 3-(décyldiméthylammonio)-propanesulfonate (Zwittergent 3-8, 3-12, 3-14), de 3-[3-(cholamidopropyl)-diméthylammonio]propanesulfonate (CHAPS), de 3-[3-(cholamidopropyl)-diméthylammonio]-2-hydroxy-1-propanesulfonate (CHAPSO), de N,N-bis[3-D-glucoamido)propyl]cholamide (Big CHAP), de sulfobétaïne détergente (C7BzO), de Tergitol (NP 7, 9, 11, 13, 15), de N-(alkyl en C₁₀ à C₁₆)-N,N-diméthylglycine bétaïne (Empigen BB), de n-dodécyl-maltoside, de Luberol PX, de n-octyl-glucoside, de 3-(4-heptyl)phényl-3-hydroxy-propyl-diméthylammonio-propane sulfonate (C7BzO), de sulfobétaïnes non détergentes et de combinaisons de ceux-ci.

21. Procédé selon la revendication 17, dans lequel l'additif stabilisant est une bétaïne.

22. Procédé selon la revendication 17, comprenant en outre le stockage des produits de la réaction de séquençage de l'ADN, du milieu de purification et de l'additif stabilisant pendant au moins huit heures sans déstabiliser les produits de la réaction de séquençage de l'ADN.
